# EUROPEAN PATENT SPECIFICATION

(11) **EP 3 010 529 B1**
(45) Date of publication and mention of the grant of the patent: **06.05.2020**
(21) Application number: 14799878.5
(22) Date of filing: 20.06.2014
(51) Int. Cl.: A61K 38/25, A61P 9/00

(54) **USE OF UNACYLATED GHRELIN, FRAGMENTS AND ANALOGS THEREOF AS ANTIOXIDANT**
VERWENDUNG VON NICHTACYLIERTEM GHRELIN SOWIE FRAGMENTEN UND ANALOGEN DAVON ALS ANTIOXIDANTIEN
UTILISATION DE GHRÉLINE NON ACÉTYLÉE, DE SES FRAGMENTS ET SES ANALOGUES COMME AGENTS ANTIOXYDANTS

(30) Priority: 21.06.2013 US 201361837723 P
(43) Date of publication of application: 27.04.2016
(73) Proprietor: Millendo Therapeutics SAS, 69130 Ecully (FR)
(72) Inventor: BRIZZI, Maria Felice, I-10133 Torino (IT); GHIGO, Ezio, I-10125 Torino (IT)
(74) Representative: BCF Global
(86) International application number: PCT/IB2014/001538
(87) International publication number: WO 2014/203074

(56) References cited:
- WO-A1-2008/145749
- WO-A2-2009/150214
- G. TOGLIATTO ET AL: "Unacylated Ghrelin Promotes Skeletal Muscle Regeneration Following Hindlimb Ischemia via SOD-2-Mediated miR-221/222 Expression", JOURNAL OF THE AMERICAN HEART ASSOCIATION, vol. 2, no. 6, 5 December 2013 (2013-12-05), pages e000376-e000376, XP055167113, DOI: 10.1161/JAHA.113.000376
- SHIMADA TAKESHI ET AL: "Des-acyl ghrelin protects microvascular endothelial cells from oxidative stress-induced apoptosis through sirtuin 1 signaling pathway", METABOLISM, CLINICAL AND EXPERIMENTAL, vol. 63, no. 4, 28 December 2013 (2013-12-28), pages 469-474, XP028657734, ISSN: 0026-0495, DOI: 10.1016/J.METABOL.2013.12.011
- G. TOGLIATTO ET AL: "Unacylated Ghrelin Rescues Endothelial Progenitor Cell Function in Individuals With Type 2 Diabetes", DIABETES, vol. 59, no. 4, 1 April 2010 (2010-04-01), pages 1016-1025, XP055167526, ISSN: 0012-1797, DOI: 10.2337/db09-0858
- RICCARDA GRANATA ET AL: "Des-Acyl Ghrelin Fragments and Analogues Promote Survival of Pancreatic [beta]-Cells and Human Pancreatic Islets and Prevent Diabetes in Streptozotocin-Treated Rats", JOURNAL OF MEDICINAL CHEMISTRY, vol. 55, no. 6, 22 March 2012 (2012-03-22) , pages 2585-2596, XP055167521, ISSN: 0022-2623, DOI: 10.1021/jm201223m

## Description

### FIELD OF TECHNOLOGY

The present invention relates to the field of oxidative stress-induced damage and the development of compositions and methods to protect against oxidative stress-induced damage, to reduce oxidative stress-induced damage and to improve resistance to oxidative stress-induced damage. The present invention also relates to the use of unacylated ghrelin fragments, in order to protect against oxidative stress-induced damage, to reduce oxidative stress-induced damage and to improve resistance to oxidative stress-induced damage.

### BACKGROUND INFORMATION

Ghrelin (also referred as "acylated ghrelin" or abbreviated as "AG") is a 28 amino acid peptide, purified and identified from rat stomach and characterized by the presence of an n-octanoyl modification on the Ser3 residue¹. Acylation of ghrelin is catalyzed by the enzyme ghrelin O-acyl transferase (GOAT). Ghrelin is the endogenous ligand of the growth hormone (GH) secretagogue receptor (GHSR-1a)^{2,3} and is now mostly recognized as a potent orexigenic factor stimulating food intake and modulating energy expenditure^{4,5,6}. At the peripheral level, ghrelin exerts probably its major physiological action regulating glucose and lipid metabolism⁷. In fact, ghrelin has a diabetogenic action⁸ and suppresses glucose-stimulated insulin secretion and deteriorates glucose tolerance⁹.

Unacylated ghrelin (also referred as "des-acyl ghrelin" or abbreviated as "UAG"), is the non-acylated form of ghrelin. Its concentration in plasma and tissue is higher compared to ghrelin. UAG has long been considered as a product with no physiological role as it fails to bind the only known ghrelin receptor GHSR-1a at physiological concentrations and has no physiological effect on GH secretion¹⁰. However, UAG is a biologically active peptide⁴⁹. It has been shown to prevent the hyperglycemic effects of ghrelin when administered concomitantly in healthy volunteers (as reported in U.S. Patent 7,825,090. This initial observation is supported by several reports on the anti-diabetogenic potential of UAG¹²⁻¹⁶. The anti-diabetogenic effects and ghrelin-antagonizing effects of UAG, fragments and analogs thereof have been reported in U.S. Patent 7,485,620; U.S. Patent 7,666,833; U.S. Patent 8,071,368; U.S. Patent 8,222,217; U.S. Patent 8,318,664; U.S. Patent 8,476,408 and in U.S. Patent Application 2010/0016226, U.S. Patent Application 2013/0157936, WO 2009/150214 and WO 2013/088241.

AG and UAG have been shown to exert effects on muscle cell and vascular cell differentiation through a common receptor^{18,19}. However, several studies have shown that UAG and AG exhibit opposing metabolic actions^{20,21,49}. Similarly, UAG and AG induce different biological responses in neonatal mouse and rat cardiomyocytes²² and only UAG protects endothelial progenitor cells (EPCs) from oxidative stress by avoiding reactive oxygen species (ROS) generation^{23,24,50} as reported in U.S. Patent Application 2010/0016226 and WO 2009/150214.

Oxidative stress plays a major role in tissue damage and is important in the development and progression of several conditions and diseases¹⁷. For example, oxidative stress is suspected to be significant in neurodegenerative diseases such as Lou Gehrig's disease, Parkinson's disease, Alzheimer's disease, and Huntington's disease. Cumulative oxidative stress with disrupted mitochondrial respiration and mitochondrial damage has been associated with Alzheimer's disease, Parkinson's disease, and other neurodegenerative diseases. Oxidative stress is also thought to be linked to certain cardiovascular disease.

Oxidative stress further plays a role in the ischemic cascade due to oxygen reperfusion injury following hypoxia (i.e., reperfusion injury). Oxidative stress has also been implicated in chronic fatigue syndrome and shown to contribute to tissue injury following irradiation and hyperoxia, as well as in diabetes.

Oxidative stress is present in peripheral arterial disease (PAD) which is a widespread condition caused by atherosclerosis of the peripheral arteries²⁵. Although surgical or endovascular intervention remains the standard therapy to improve blood flow²⁶, even after successful revascularisation, most patients complain of persistent or recurring symptoms²⁷. Changes in local oxygen availability in PAD result in increased numbers of dysfunctional mitochondria^{28,29}. Defective mitochondrial electron transfer chain and increased ROS generation are important determinants of oxidative stress-induced damage and impaired cellular functions³⁰⁻³³ that ultimately lead to muscle damage³⁴. Interestingly, superoxide dismutase-2 (SOD-2), the initial line of defense against ROS in the mitochondria, is deficient in PAD muscles²⁸. Consistently, anti-oxidant administration ameliorates skeletal muscle mitochondrial dysfunction and functional recovery in humans³⁵.

In an aging population with an increasingly high incidence of metabolic diseases, new treatment options for circumventing the damages caused by oxidative stress represents a major unmet need. The earlier observation that UAG, fragments and analogs thereof protect endothelial progenitor cells (EPCs) from diabetes-associated oxidative stress by avoiding AGE-induced ROS generation^{23,24,50} as led to evaluate the protective effect of UAG as an antioxidant.

### SUMMARY OF THE INVENTION

According to one aspect, the present invention relates to a fragment of unacylated ghrelin and/or pharmaceutically acceptable salts thereof wherein the fragment consists of amino acid sequence SEQ ID NO: 6 and/or pharmaceutically acceptable salts thereof, for its use in protecting a subject against reperfusion injury; particularly when the subject suffers from ischemia, or suffers from a stroke, or for protecting a subject from reperfusion injury following organ transplantation or for preventing ischemia-reperfusion injury in the subject.

Particularly, the UAG fragment for the use of the invention modulates cellular levels of superoxide-dismutase-2 (SOD-2). The modulation of cellular levels of SOD-2 includes increasing cellular levels or expression of SOD-2.

### BRIEF DESCRIPTION OF THE FIGURES

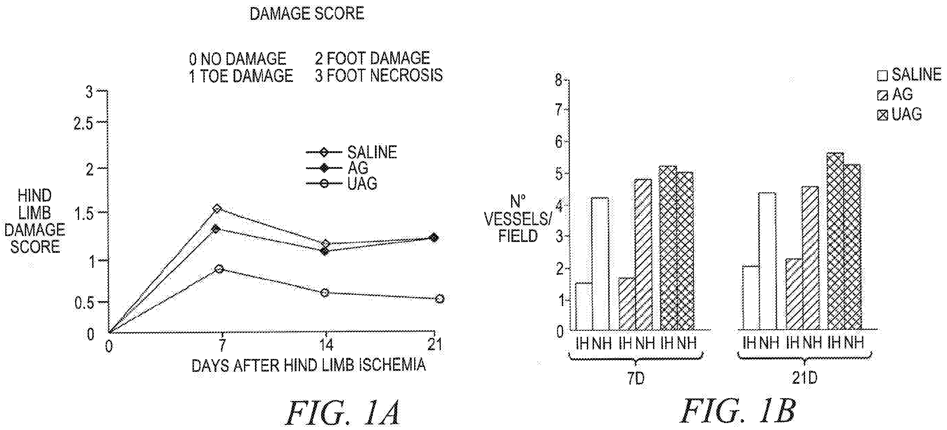

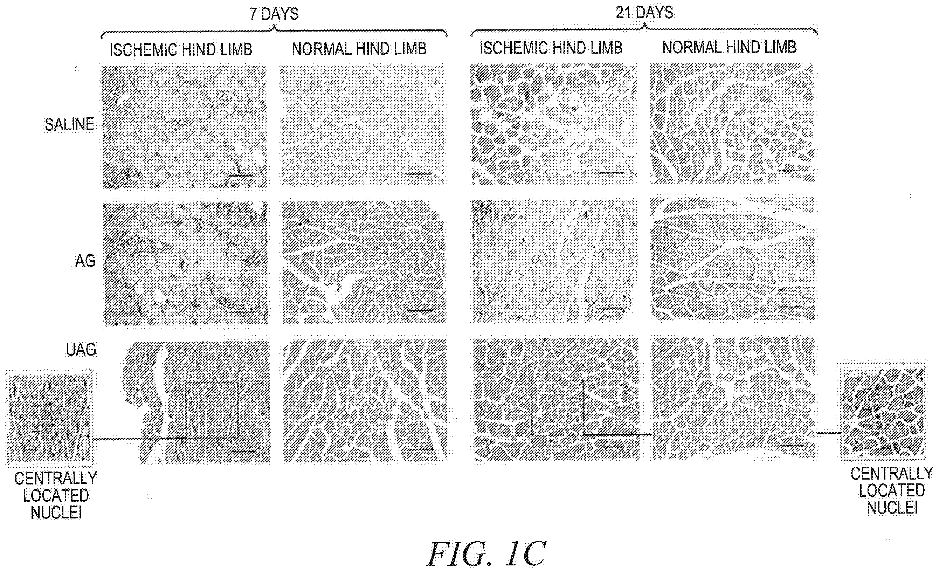

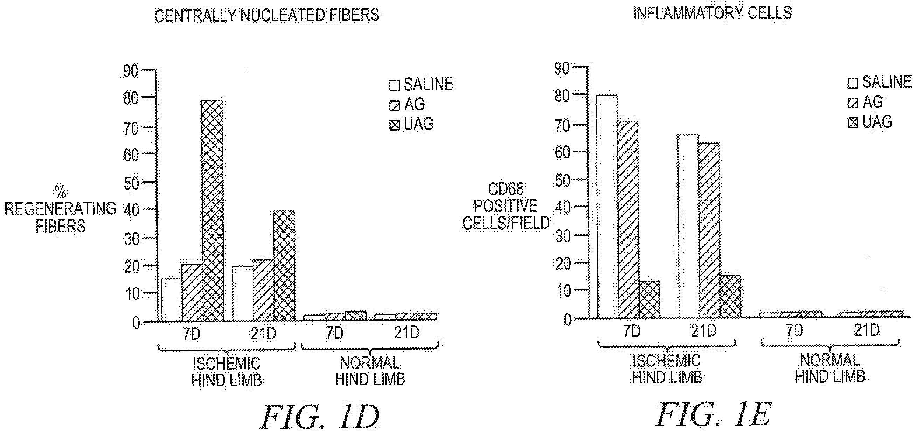

Figures 1A to 1E (Background Illustration) illustrate the protective effect of UAG on ischemia-mediated functional impairment in skeletal muscle. In Figure 1A, foot damage score was evaluated for the indicated times. In Figure 1B, the number of vessels in ischemic (ih) and normo-perfused (nh) gastrocnemius muscles of each group of animals was evaluated. In Figure 1C, sections of ischemic and normo-perfused (normal) muscles from UAG, AG and saline mice were stained. Insets show myofibers at higher magnification; *black arrows* indicate regenerating myofibers, characterized by central nucleus location. In Figure 1D, the percentage of regenerating fibers was quantified and characterized by the presence of centrally located nucleus. In Figure 1E, inflammatory cells in ischemic and normal muscles of UAG, AG and saline mice were quantified.

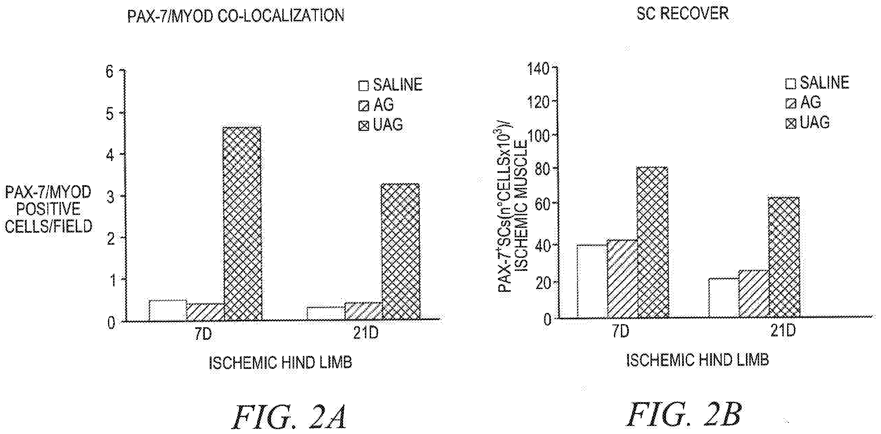

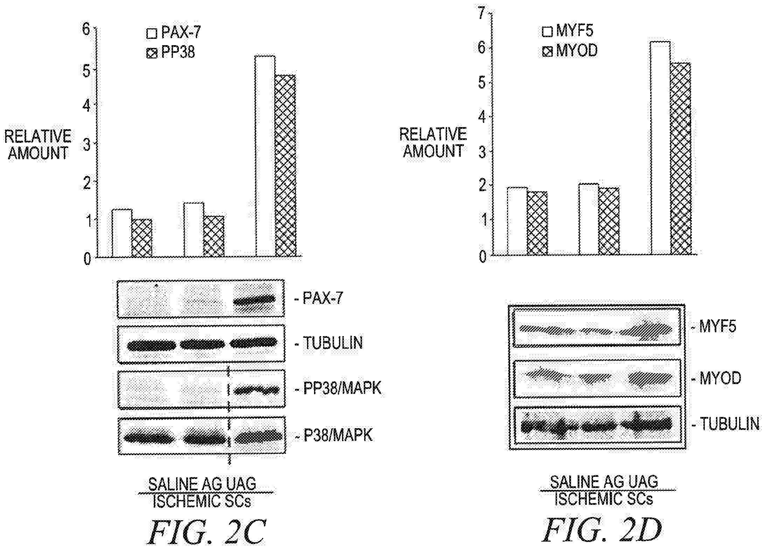

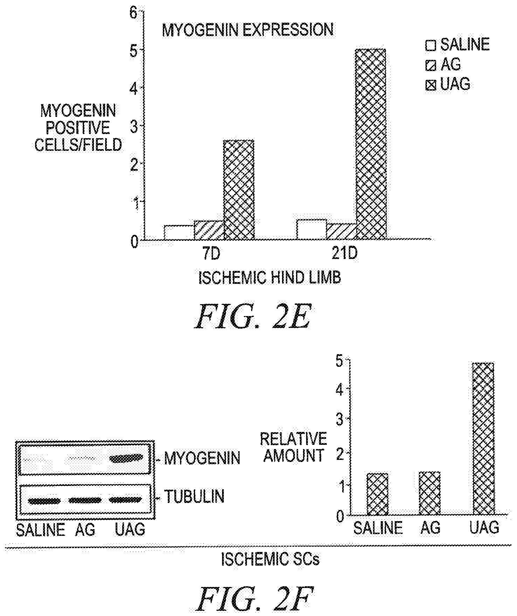

Figures 2A to 2F (Background Illustration) illustrate that UAG improves SMR and SC cell-cycle entry via p38/MAPK phosphorylation. In Figure 2A, Pax-7+/MyoD+ cells in ischemic muscles were quantified. In Figure 2B, the number of SCs from ischemic muscles of treated mice was calculated. In Figures 2C and 2D, cell extracts from SCs recovered from ischemic muscles were analyzed by Western blot for Pax-7 and phospho(p)-p38/MAPK (C) or for Myf5 and MyoD content (D). In Figure 2E, sections of ischemic muscles recovered from treated mice were stained for myogenin and DAPI and myogenin+ cells in ischemic limb of treated mice were quantified. In Figure 2F, the myogenin content was evaluated by Western blot in SCs from ischemic muscles of treated animals.

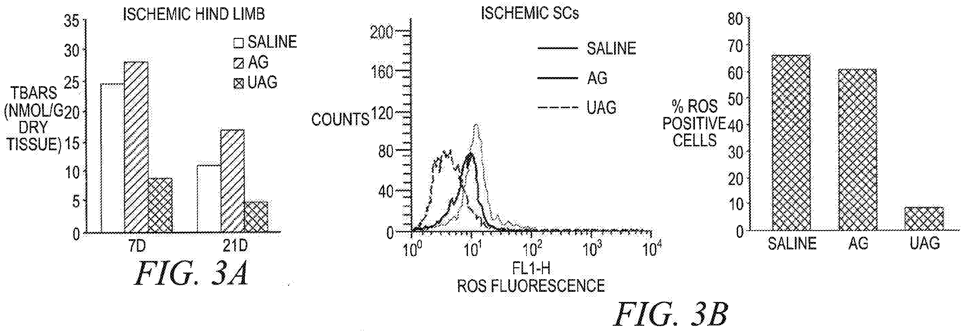

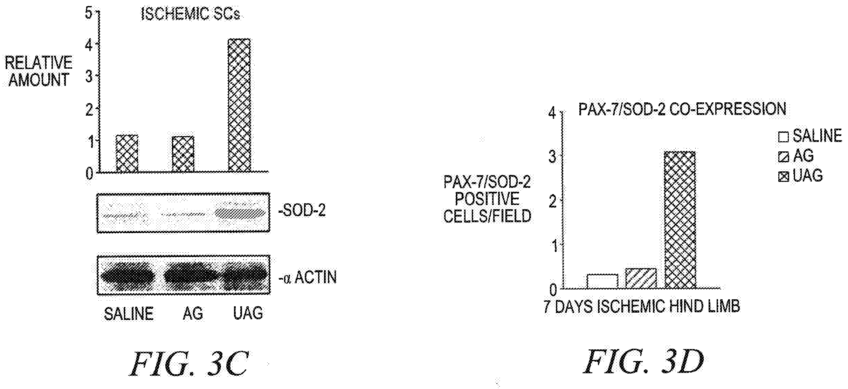

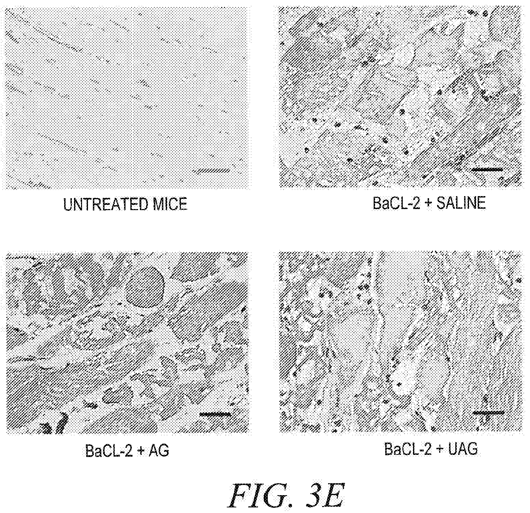

Figures 3A to 3E (Background Illustration) illustrate that UAG prevents ROS production in SCs by inducing SOD-2 expression. In Figure 3A, TBARS were determined in gastrocnemius muscle of UAG, AG and saline mice. In Figure 3B, ROS generation was evaluated by DCF-DA assay on SCs recovered from muscles of UAG, AG and saline mice. In Figure 3C, SCs recovered from ischemic muscles of treated mice were subjected to Western blot normalized; SOD-2 content was evaluated. In Figure 3D, representative sections of muscles recovered at day 7 after ischemia were stained (Pax-7, SOD-2 and DAPI staining) and Pax-7/SOD-2 positive cells in ischemic muscles of treated mice were quantified. Figure 3E shows representative H&E stained sections of toxic damage induced by injection of 1% barium chloride (BaCl2) in gastrocnemius muscles of C57BL/6J mice.

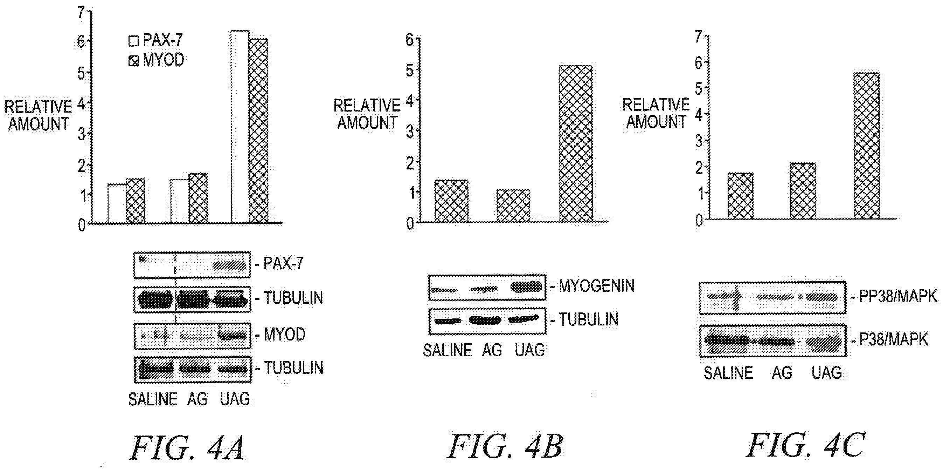

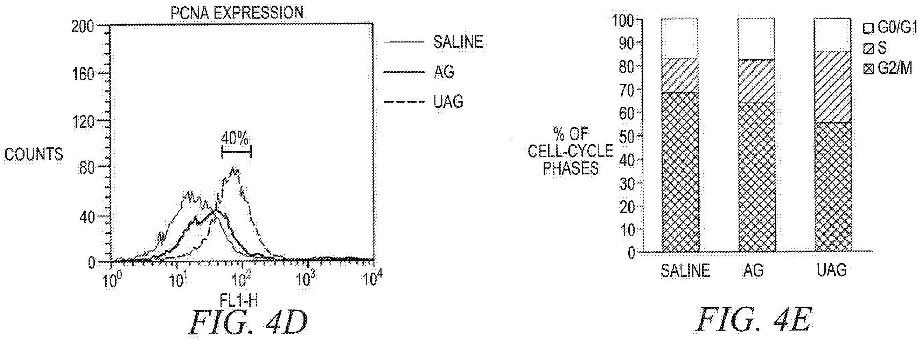

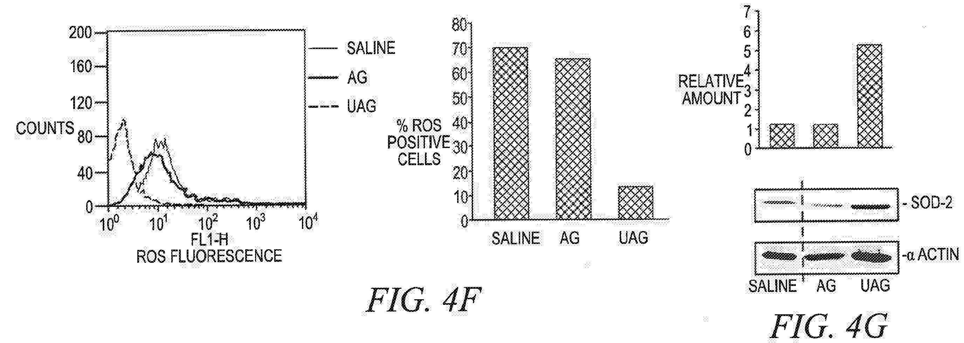

Figures 4A to 4G (Background Illustration) illustrate the *in vitro* effects of UAG on primary SCs. In Figures 4A, 4B and 4C, SCs recovered from normoperfused muscles were subjected to *in vitro* ischemia in presence of the indicated stimuli. Cell extracts were analyzed by Western blot for Pax-7 and MyoD (A), for myogenin (B) and for pp38/MAPK content (C) by densitometry. In Figure 4D, SCs subjected to *in vitro* ischemia and treated as indicated were analyzed by FACS analysis for PCNA expression. In Figure 4E, FACS analysis indicates the percentage of SCs, treated as above, in the different cell-cycle phases. In Figure 4F, ROS generation was evaluated by DCF-DA assay performed on SCs subjected to *in vitro* ischemia and treated as indicated. In Figure 4G, SOD-2 content was analyzed by Western blot in SCs subjected to *in vitro* ischemia.

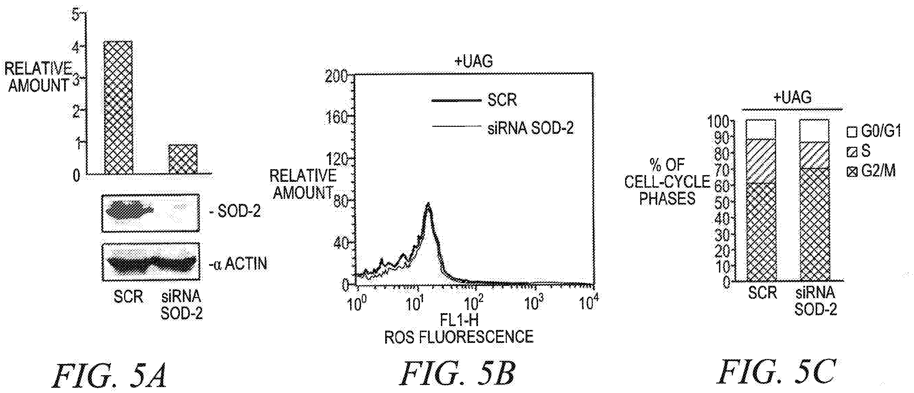

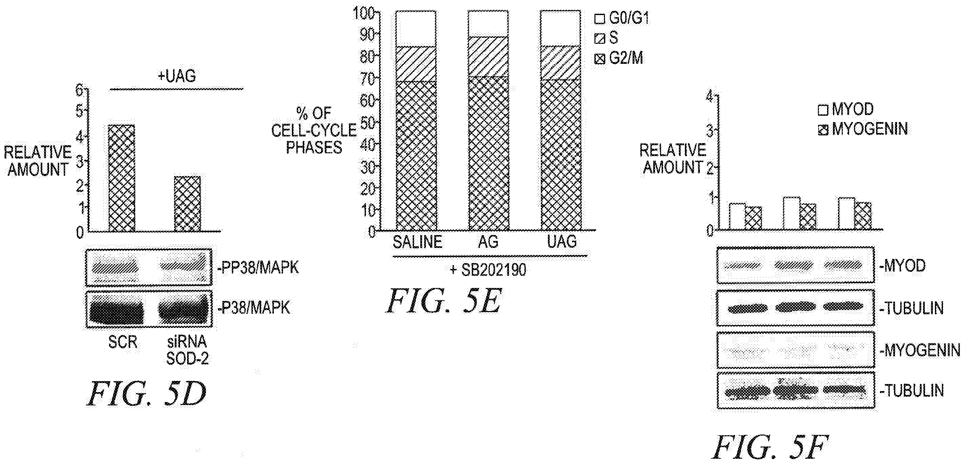

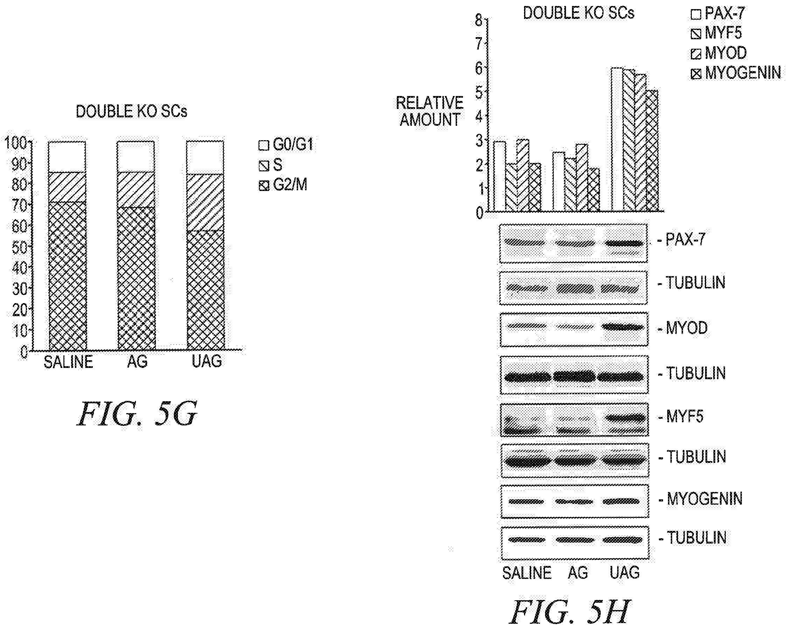

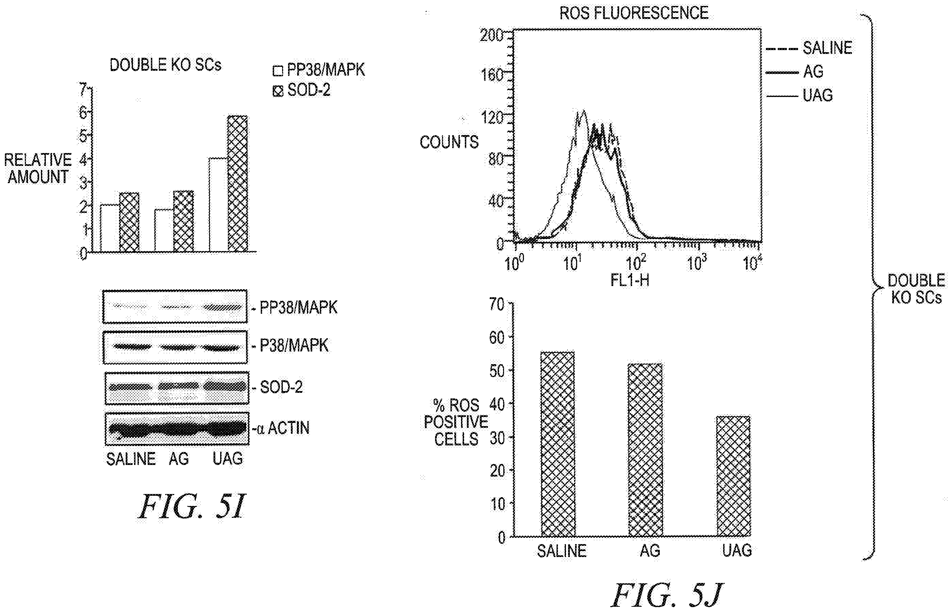

Figures 5A to 5J (Background Illustration) illustrate that UAG induces SC cell-cycle entry via SOD-2 and p38/MAPK phosphorylation. In Figure 5A, SOD-2 content was evaluated in SCs transfected for 48h with scramble or SOD-2 siRNA. In Figure 5B, ROS generation was evaluated by DCF-DA assay performed on SCs treated as indicated. In Figure 5C, FACS analysis indicates the percentage of SCs transfected with scramble or with SOD-2 siRNA in presence of UAG in the different cell-cycle phases. In Figure 5D, SCs transfected with scramble or SOD-2 siRNA and stimulated with UAG were analyzed by Western blot for p-p38/MAPK content by densitometry. In Figure 5E, FACS analysis indicates the percentage of SCs in the different cell-cycle phases following 24h treatment with the indicated stimuli. In Figure 5F, cell extracts from SCs treated as indicated were analyzed by Western blot for MyoD and myogenin content by densitometry. In Figure 5G, SCs recovered from double KO mice were stimulated with saline, AG and UAG and subjected to *in vitro* ischemia. FACS analysis was performed to evaluate SC cell-cycle progression. In Figures 5H and 5I cell extracts from KO-derived SCs, treated as indicated and subjected to *in vitro* ischemia were analysed by western blot for Pax-7, MyoD, Myf5 and myogenin (H) and for p-p38/MAPK and SOD-2 (I) content by densitometry. In Figure 5J, ROS generation was evaluated by DCF-DA assay performed on SCs derived from double KO mice treated as indicated.

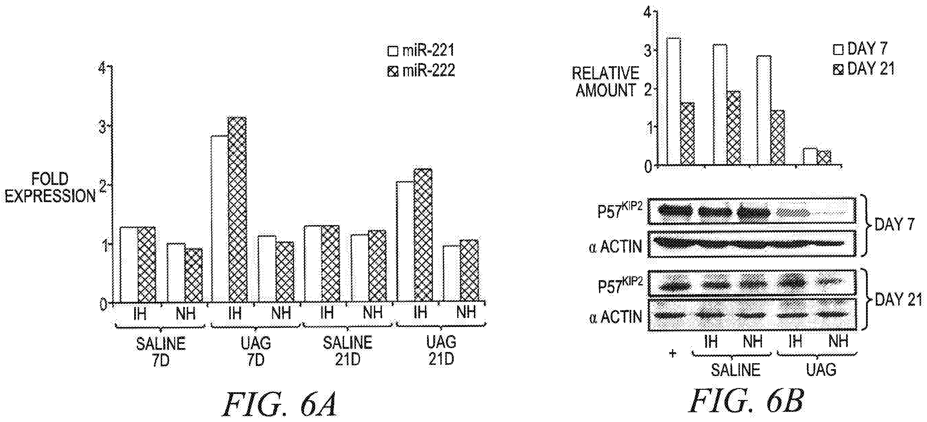

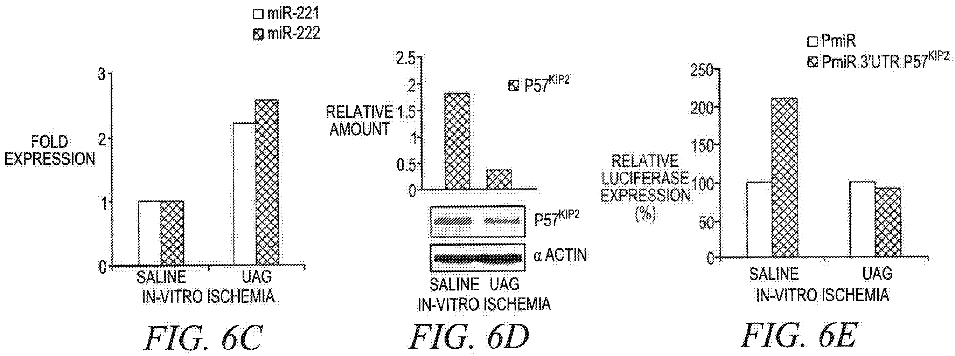

Figures 6A to 6E illustrate that UAG induces SC cell-cycle entry by regulating miR-221/222 expression. In Figure 6A, miR-221/222 expression was evaluated by qRT-PCR on SCs from ih and nh muscles of mice treated as indicated. In Figure 6B, p57^{kip2} content was analyzed in SCs from ih and nh muscles by densitometry. In Figure 6C, miR-221/222 expression was analyzed by qRT-PCR on SCs from nh muscles, subjected to *in vitro* ischemia and treated as indicated. In Figure 6D, cell extracts from SCs treated as above were analyzed for p57^{kip2} content. In Figure 6E, SCs were transfected with pmiR or pmiR-3'UTR p57^{kip2} luciferase constructs, treated as indicated and subjected to *in vitro* ischemia.

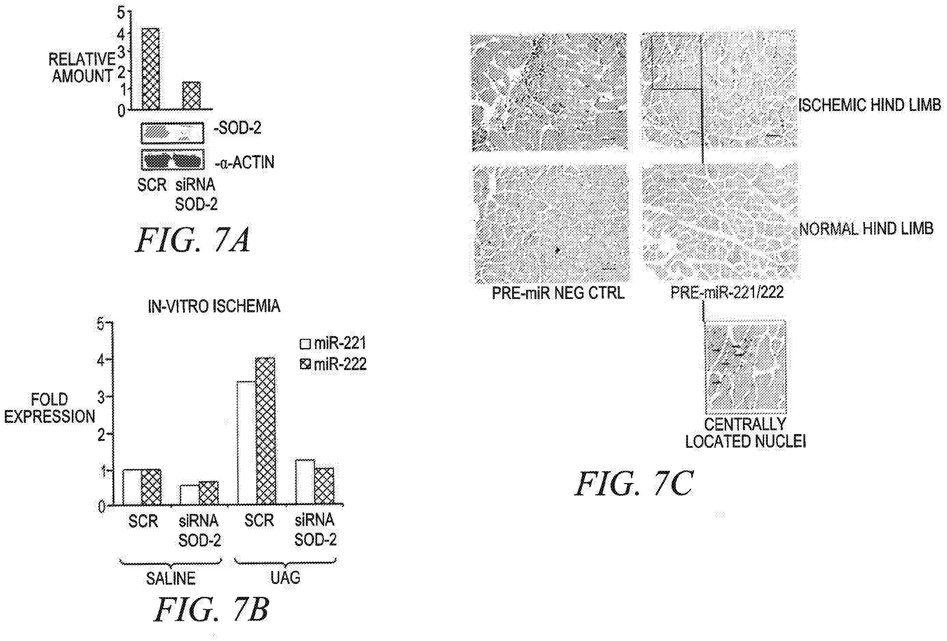

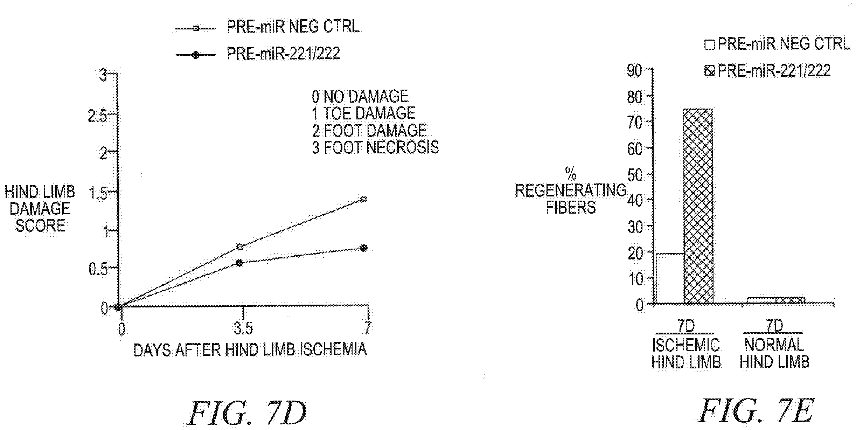

Figures 7A to 7E (Background Illustrtation) illustrate the *in vivo* effect of UAG on miR221-222 expression. In Figure 7A, SOD-2 content in primary SCs, recovered from normo-perfused muscles and transfected for 48h with the scramble or with the SOD-2 siRNA was analyzed. In Figure 7B, miR-221/222 expression was evaluated by qRT-PCR on SCs silenced for SOD-2 and subjected to *in vitro* ischemia. Figure 7C presents representative H&E stained sections of ischemic and normo-perfused (normal) muscles of mice injected with pre-miR negative control (neg ctrl) or with pre-miR221/222. Inset shows myofibers at higher magnification; *black arrows* indicate regenerating myofibers, characterized by central nucleus location. In Figure 7D, foot damage score of treated mice was evaluated for the indicated times. In Figure 7E, percentage of regenerating fibers in pre-miR neg ctrl or pre-miR-221/222-treated mice after ischemia was obtained.

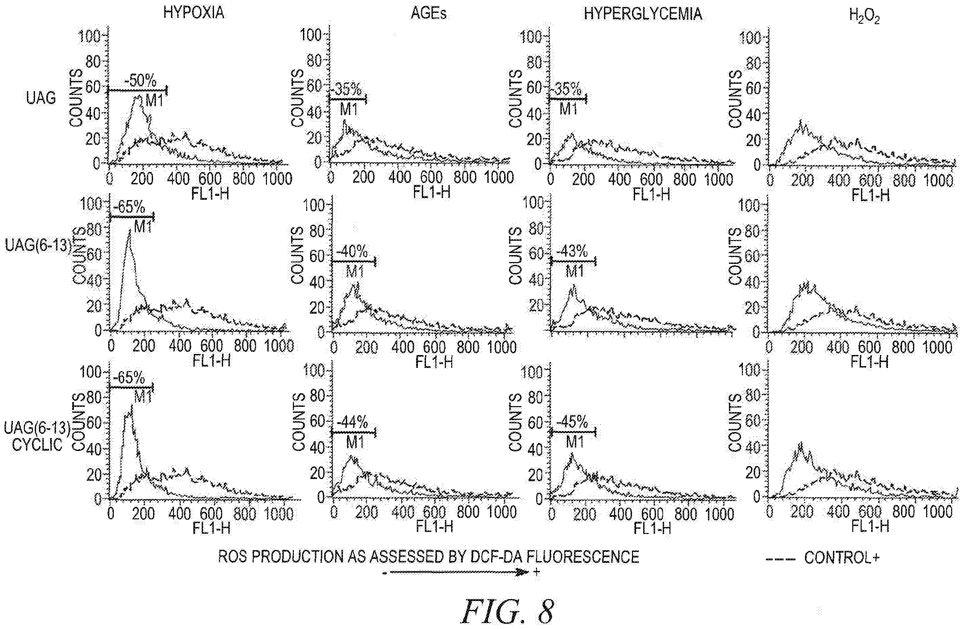

Figure 8 illustrates the protective effect of UAG and a fragment thereof on C2C12 mouse muscular cell line against oxidative stress. The effects of ROS production on C2C12 cells treated with UAG (comparative experiment), UAG fragment (UAG (6-13)) and UAG cyclic fragment (UAG (6-13)cyclic) were assessed. Oxidative stress was induced by hypoxia (1% O₂), hyperglycemia (25 mM), advanced glycation end-products (AGEs) or H₂O₂.

### DETAILED DESCRIPTION

For ease of reference, the following abbreviations and designations are used herein throughout:
- AG: ghrelin or acylated ghrelin
- UAG: unacylated ghrelin or des-acyl ghrelin
- UAG (6-13): unacylated ghrelin having residues 6 to 13 of SEQ ID NO: 1
- GHSR: growth hormone secretagogue receptor
- ROS: reactive oxygen species
- RNS: reactive nitrogen species
- PAD: peripheral arterial disease
- SOD: superoxide dismutase
- SMR: skeletal muscle regeneration
- SC: satellite cell
- EPC: endothelial progenitor cell
- AGE: advanced glycation end product
- FACS: florescence-activated cell sorting
- e.g.: for example

Unless defined otherwise, all technical and scientific terms used herein have the same meaning as commonly understood by one of skill in the art to which the invention pertains.

The invention defined in the present application stems from, but is not limited to, the unexpected findings by the inventors that UAG induces muscle regeneration after ischemia by reducing ROS-mediated muscle damage via a mechanism involving SOD-2 and miR221-222.

The data presented herein therefore provides the first evidence of the involvement of UAG, fragments thereof and analogs thereof in the primary enzymatic antioxidant defense against oxidative stress and ROS production and suggests the therapeutic potential of UAG, fragments thereof and analogs thereof in the treatment of conditions in which ROS scavenging and antioxidant efficiency is required.

The expressions "unacylated ghrelin", "des-acyl ghrelin" and the abbreviation "UAG" are intended to mean peptides that have the amino acid sequence specified in SEQ ID NO: 1 which amino acid sequence is:

Unacylated ghrelin may also be referred to as UAG (1-28).

Naturally-occurring variations of UAG include peptides that contain substitutions, additions or deletions of one or more amino acids which result due to discrete changes in the nucleotide sequence of the encoding ghrelin gene or alleles thereof or due to alternative splicing of the transcribed RNA. It is understood that the changes do not substantially affect the properties, pharmacological and biological characteristics of unacylated ghrelin variants. Those peptides may be in the form of salts. Particularly the acidic functions of the molecule may be replaced by a salt derivative thereof such as, but not limited to, a trifluoroacetate or an acetate salt. By "peptide", "polypeptide" or "protein" is meant any chain of amino acids, regardless of length or post-translational modification (e.g., glycosylation or phosphorylation), or chemical modification, or those containing unnatural or unusual amino acids such as D-Tyr, ornithine, amino-adipic acid. The terms are used interchangeably in the present application.

The expressions "fragments" and "fragments thereof" refer to amino acid fragments of a peptide such as UAG. Fragments of UAG are shorter than the amino acid sequence depicted in SEQ ID NO: 1, therefore are shorter than 28 amino acid residues. Fragments of UAG may be 27, 26, 25, 24, 23, 22, 21, 20, 19, 18, 17, 16, 15, 14, 13, 12, 11, 10, 9, 8, 7, 6, 5 or 4 amino acid residues in length. For example, fragments of UAG may have the amino acid sequences depicted in Table 1 below:

**Table 1: UAG fragments**

| **Fragment** | **SEQ ID NO:** | **Amino Acid Sequence** |
|---|---|---|
| UAG (1-14) | 2 | Gly-Ser-Ser-Phe-Leu-Ser-Pro-Glu-His-Gln-Arg-Val-Gln-Gln |
| UAG (1-18) | 3 | Gly-Ser-Ser-Phe-Leu-Ser-Pro-Glu-His-Gln-Arg-Val-Gln-Gln-Arg-Lys-Glu-Ser |
| UAG (1-5) | 4 | Gly-Ser-Ser-Phe-Leu |
| UAG (17-28) | 5 | Glu-Ser-Lys-Lys-Pro-Pro-Ala-Lys-Leu-Gln-Pro-Arg |
| UAG (6-13) | 6 | Ser-Pro-Glu-His-Gln-Arg-Val-Gln |
| UAG (8-13) | 7 | Glu-His-Gln-Arg-Val-Gln |
| UAG (8-12) | 8 | Glu-His-Gln-Arg-Val |
| UAG (6-18) | 9 | Ser-Pro-G lu-His-Gln-Arg-Val-Gln-Gln-Arg-Lys-Glu-Ser |
| UAG (8-11) | 10 | Glu-His-Gln-Arg |
| UAG (9-12) | 11 | His-Gln-Arg-Val |
| UAG (9-11) | 29 | His-Gln-Arg |
| UAG (14-1) | 30 | Gln Gln Val Arg Gin His Glu Pro Ser Leu Phe Ser Ser Gly |

The fragment for its use according to the invention consists of amino acid sequence SEQ ID NO: 6 (UAG (6-13) and/or pharmaceutically acceptable salts thereof.

In one embodiment of the present invention, as the UAG fragment is used in a form that is "purified", "isolated" or "substantially pure". The peptides are "purified", "isolated" or "substantially pure" when they are separated from the components that naturally accompany them. Typically, a compound is substantially pure when it is at least 60%, 65%, 70%, 75%, 80%, 85%, 90%, 91%, 92%, 93%, 94%, 95%, 96%, 97%, 98%, or 99%, by weight, of the total material in a sample.

The expressions "biological activity" or "biological property", or the term "activity" in reference to the peptides as defined herein, are used interchangeably herein and refer to the biological, cellular and/or pharmaceutical abilities of the peptides as defined herein and include, but are not limited to, the capacity of replacing UAG in the biological functions of UAG as described in U.S. Patents 7,485,620; 7,666,833; 8,071,368; 7,825,090; 8,222,217; 8,318,666 and 8,476,408 and in U.S. Patent Applications 2010/0016226 and 2013/0157936 or as described in the present application, such as, but not limited to, in protecting against oxidative stress-induced damage, reducing oxidative stress-induced damage, protecting against cell injuries induced by oxidative stress, protecting against ROS-induced cell injuries, inducing muscle regeneration, reducing functional impairment of muscle cells, inducing skeletal muscle regeneration, having antioxidant effect on oxidative-damaged cells, reducing functional impairment of skeletal muscle cells, protecting satellite cells from oxidative stress-induced damage and modulating cellular levels of SOD-2.

As used herein, the term "modified" refers to any changes made to a given peptide, such as changes to the length of the peptide, the amino acid sequence, chemical structure, co-translational modification, or post-translational modification of a peptide.

The term "post-translational modification" refers to any modification of a natural or non-natural amino acid that occurs to such an amino acid after it has been incorporated into a peptide chain. The term encompasses, by way of example only, co-translational *in vivo* modifications, co-translational *in vitro* modifications (such as in cell-free translation system), post-translational *in vivo* modifications, and post-translational *in vitro* modifications. Examples of post-translational modifications are, but are not limited to, glycosylation, acetylation, acylation, amidation, carboxylation, phosphorylation, PEGylation, addition of salts, amides or esters, in particular C-terminal esters, and N-acyl derivatives of the peptides as defined herein. The types of post-translational modifications are well known.

The peptide for its use according to the present invention may also be in cyclic form, such that the N- or C-termini are linked head-to-tail either directly, or through the insertion of a linker moiety, such moiety itself generally comprises one or more amino acid residues as required to join the backbone in such a manner as to avoid altering the three-dimensional structure of the peptide with respect to the non-cyclic form. Such peptide derivatives may have improved stability and bioavailability relative to the non-cyclic peptides. A prefered cyclic peptide of the present invention is cyclic UAG (6-13) of SEQ ID NO 25.

Methods for cyclizing peptides are well known in the art and for example may be accomplished by disulfide bond formation between two side chain functional groups, amide or ester bond formation between one side chain functional group and the backbone α-amino or carboxyl function, amide or ester bond formation between two side chain functional groups, or amide bond formation between the backbone α-amino and carboxyl functions. These cyclization reactions have been traditionally carried out at high dilution in solution. Cyclization is commonly accomplished while the peptide is attached to the resin. One of the most common ways of synthesizing cyclic peptides on a solid support is by attaching the side chain of an amino acid to the resin. Using appropriate protection strategies, the C-and N-termini can be selectively deprotected and cyclized on the resin after chain assembly. This strategy is widely used, and is compatible with either tert-butyloxycarbonyl (Boc) or 9-fluorenylmethoxycarbonyl (Fmoc) protocols. However, it is restricted to peptides that contain appropriate side chain functionality to attach to the solid support. A number of approaches may be used to achieve efficient synthesis of cyclic peptides. One procedure for synthesizing cyclic peptides is based on cyclization with simultaneous cleavage from the resin. After an appropriate peptide sequence is assembled by solid phase synthesis on the resin or a linear sequence is appended to resin, the deprotected amino group can react with its anchoring active linkage to produce protected cyclic peptides. In general, a final deprotection step is required to yield the target cyclic peptide.

Lactamazation, a form of cyclization, may be performed to form a lactam bridge using Fmoc synthesis, amino acids with different protecting groups at the lateral chains may be introduced, such as, but not limited to, aspartic acid (or glutamic) protected with allyl ester at the beta ester (or gamma ester for glutamic acid) and lysine protected with allyloxy carbamate at the N-ε. At the end of the synthesis, with the N-terminus of the peptide protected with Fmoc, Boc or other protecting group different from Alloc, the allyl and alloc protecting groups of aspartic acid and lysine may be deprotected with, for example, palladium (0) followed by cyclization using PyAOP (7-Azabenzotriazol-1-yloxytris(pyrrolidino) phosphonium-hexafluorophosphate) to produce the lactam bridge.

Unless otherwise indicated, an amino acid named herein refers to the L-form. Well recognized abbreviations in the art will be used to describe amino acids, including levorotary amino acids (L-amino acids or L or L-form) and dextrorotatory amino acids (D-amino acids or D or D-form), Alanine (Ala or A), Arginine (Arg or R), Asparagine (Asn or N), Aspartic acid (Asp or D), Cysteine (Cys or C), Glutamic acid (Glu or E), Glutamine (Gln or Q), Glycine (Gly or G), Histidine (His or H), Isoleucine (Ile or I), Leucine (Leu or L), Lysine (Lys or K), Methionine (Met or M), Phenylalanine (Phe or F), Proline (Pro or P), Serine (Ser or S), Threonine (Thr or T), Tryptophan (Trp or W), Tyrosine (Tyr or Y) and Valine (Val or V). An L-amino acid residue within the native peptide sequence may be altered to any one of the 20 L-amino acids commonly found in proteins or any one of the corresponding D-amino acids, rare amino acids, such as, but not limited to, 4-hydroxyproline or hydroxylysine, or a non-protein amino acid, such as P-alanine or homoserine.

The UAG fragments for their use of the invention may also be part of a fusion protein. It is often advantageous to include an additional amino acid sequence such as a signal sequence or a leader sequence which contains for example secretory sequences, pro-sequences, linker sequences. Some of these additional sequences may aid in purification such as multiple histidine residues (HA-tag) or an additional sequence for stability during recombinant production. Some of these additional sequences may aid in directing the peptides as defined herein to a specific target in an organism such as in targeting the peptides as defined herein to a specific organ or tissue or targeting the peptides as defined herein to a specific organelle within a cell.

In some implementations, the UAG fragment may be in a protein precursor format (i.e., pro-UAG fragment, pre-pro-UAG fragment). In some implementations, a leader sequence may be attached to target the peptide as defined herein to the mitochondrial. In this implementation, the leader sequence is a mitochondrial leader sequence. Mitochondrial leader sequences are well known in the art.

The additional amino acids or sequence may be linked to at the N-terminal or at the C-terminal of the peptide or may be linked to any amino acid of the sequences located between the N- and the C-terminal to give rise the UAG fragment having a linker moiety.

The peptide for its use according to the present invention may be chemically synthesized by any of the methods well known in the art. Suitable methods for synthesizing the protein include, for example those described by Stuart and Young in "Solid Phase Peptide Synthesis" Second Edition, Pierce Chemical Company (1984), and in "Solid Phase Peptide Synthesis" Methods Enzymol. 289, Academic Press, Inc, New York (1997). General methods and synthetic strategies used in providing functional and structural UAG fragments are commonly used and well known in the art and are described in publications such as: "Peptide synthesis protocols" ed, M.W. Pennigton & B. M. Dunn. Methods in Molecular Biology. Vol 35. Humana Press, NJ.,1994; "Solid phase peptide synthesis" by Stewart and Young, W. h Freeman & Co., San Francisco, 1969 and Erickson and Merrifield; and "The Proteins" Vol. 2, p. 255 et seq. (Ed. Neurath and Hill), Academic Press, New York, 1976.

The peptides as defined herein may be prepared in any suitable methods as known in the art. Such peptides include isolated naturally occurring peptides, recombinantly produced peptides, synthetically produced peptides, or peptides produced by a combination of these methods. Means and methods for preparing such peptides are well known in the art.

In the present invention, the UAG fragment and/or pharmaceutically acceptable salts thereof as defined above is for use in protecting a subject against reperfusion injury; particularly when the subject suffers from ischemia, or suffers from a stroke, or for protecting a subject from reperfusion injury following organ transplantation or for preventing ischemia-reperfusion injury in the subject.

A subject in need thereof can be any mammal, including, for example, farm animals, such as sheep, pigs, cows, and horses; pet animals, such as dogs and cats; laboratory animals, such as rats, mice and rabbits. In a preferred implementation, the subject is a human.

A subject in need thereof may be undergoing reperfusion. As used herein, the term "reperfusion" refers to the restoration of blood flow to any organ or tissue in which the flow of blood is decreased or blocked. The restoration of blood flow during reperfusion leads to respiratory burst and formation of free radicals. Decreased or blocked blood flow may be due for example, to hypoxia or ischemia. The loss or severe reduction in blood supply during hypoxia or ischemia may, for example, be due to thromboembolic stroke, coronary atherosclerosis, or peripheral vascular disease. For instance, cardiac muscle ischemia or hypoxia is commonly caused by atherosclerotic or thrombotic blockages which lead to the reduction or loss of oxygen delivery to the cardiac tissues by the cardiac arterial and capillary blood supply. Such cardiac ischemia or hypoxia may cause pain and necrosis of the affected cardiac muscle, and ultimately may lead to heart failure. Ischemia or hypoxia in skeletal muscle or smooth muscle may arise from similar causes. For example, ischemia or hypoxia in intestinal smooth muscle or skeletal muscle of the limbs may also be caused by atherosclerotic or thrombotic blockages.

The peptide for the use of the present invention include the step of administering an effective amount of UAG fragment as defined herein to the subject in need of such administration. The peptides as defined herein are administered to a subject in an amount effective in protecting from oxidative stress-induced damage. The effective amount is determined during pre-clinical trials and clinical trials by methods known in the art.

As used herein, the term "treatment" refers to both therapeutic treatments as well as to prophylactic measures. Those in need of treatment include those already with the disorder, disease or condition as well as those in which the disease, disorder or condition is to be prevented. Those in need of treatment are also those in which the disorder, disease or condition has occurred and left after-effects or scars. Treatment also refers to administering a therapeutic substance effective to improve or ameliorate, diminish symptoms associated with a disease, a disorder or a condition to lessen the severity of or cure the disease, disorder or condition, or to prevent the disease, disorder or condition from occurring or reoccurring.

For the uses of the invention, the peptides as defined herein may be formulated for, but not limited to, intravenous, subcutaneous, transdermal, topical, oral, buccal, sublingual, nasal, inhalation, pulmonary, or parenteral administration according to conventional methods. Intravenous injection may be by bolus or infusion over a conventional period of time. The peptides as defined herein may also be administered directly to a target site within a subject e.g., by biolistic delivery to an internal or external target site or by catheter to a site in an artery. The peptides can be injected directly into coronary artery during, for example, angioplasty or coronary bypass surgery, or applied onto coronary stents. Other routes of administration include intracerebroventricularly or intrathecally.

In one implementation of this embodiment, the peptides as defined herein are administered as a bolus. Accordingly, the medicament is administered as a bolus prior to meal, wherein the bolus comprises an effective amount of UAG fragment of a salt thereof. The bolus may be administered one, twice, three times or more daily or may be administered according to other dosage regimens.

Suitable dosage regiments are determined taking into account factors well known in the art such as, but not limited to, type of subject being dosed, the age, the weight, the sex and the medical condition of the subject, the route of administration, the desired affect, etc.

Active ingredients, such as the peptides as defined herein, may be administered orally as a suspension and can be prepared according to techniques well known in the art of pharmaceutical formulation and may contain, but not be limited to, microcrystalline cellulose for imparting bulk, alginic acid or sodium alginate as a suspending agent, methylcellulose as a viscosity enhancer, and sweeteners/flavoring agents. As immediate release tablets, these compositions may contain, but are not limited to microcrystalline cellulose, dicalcium phosphate, starch, magnesium stearate and lactose and/or other excipients, binders, extenders, disintegrants, diluents and lubricants. The active ingredients may be administered by way of a controlled-release delivery system.

Administered by nasal aerosol or inhalation formulations may be prepared, for example, as solutions in saline, employing benzyl alcohol or other suitable preservatives, absorption promoters to enhance bioavailability, employing fluorocarbons, and/or employing other solubilizing or dispersing agents.

The peptides as defined herein may be administered in intravenous (both bolus and infusion), intraperitoneal, subcutaneous, topical with or without occlusion, or intramuscular form. When administered by injection, the injectable solution or suspension may be formulated using suitable non-toxic, parenteral-acceptable diluents or solvents, well known in the art.

The peptides as defined herein may also be formulated for topical administration. The term "topical" as used herein includes any route of administration that enables the compounds to line the skin or mucosal tissues.

The formulation suitable for topical application may be in the form of, for example, cream, lotion, solution, gel, ointment, paste, plaster, paint, bioadhesive, or the like, and/or may be prepared so as to contain liposomes, micelles, microparticles and/or microspheres. The formulation may be aqueous, i.e., contain water, or may be nonaqueous and optionally used in combination with an occlusive overlayer so that moisture evaporating from the body surface is maintained within the formulation upon application to the body surface and thereafter.

Ointments, as is well known in the art of pharmaceutical formulation, are semisolid preparations that are typically based on petrolatum or other petroleum derivatives.

Formulations may also be prepared with liposomes, micelles, microparticles and/or microspheres. Liposomes are microscopic vesicles having a lipid wall comprising a lipid bilayer, and can be used as drug delivery systems. Micelles are known in the art to be comprised of surfactant molecules arranged so that their polar head groups form an outer spherical shell, while the hydrophobic, hydrocarbon chains are oriented towards the center of the sphere, forming a core. Microparticles are particulate carrier systems in the micron size range, normally prepared with polymers, which can be used as delivery systems for drugs or vaccines that are usually trapped within the particles. Microspheres, similarly, may be incorporated into the present formulations and drug delivery systems. Like liposomes and micelles, microspheres essentially encapsulate a drug or drug-containing formulation. Microspheres are generally, although not necessarily, formed from synthetic or naturally occurring biocompatible polymers, but may also be comprised of charged lipids such as phospholipids.

Preparations of formulations suitable for topical administration are well known in the art and described in the pertinent texts and literature.

In general, pharmaceutical compositions will comprise at least one of the peptides as defined herein together with a pharmaceutically acceptable carrier which will be well known to those skilled in the art. The compositions may further comprise for example, one or more suitable excipients, diluents, fillers, solubilizers, preservatives, stabilizers, carriers, salts, buffering agents and other materials well known in the art depending upon the dosage form utilized. Methods of composition are well known in the art.

In the present context, the term "pharmaceutically acceptable carrier" is intended to denote any material, which is inert in the sense that it substantially does not have any therapeutic and/or prophylactic effect *per se* and that are non-toxic. A pharmaceutically acceptable carrier may be added to the peptides as defined herein with the purpose of making it possible to obtain a pharmaceutical composition, which has acceptable technical properties.

Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and PEG. Carriers for topical or gel-based forms of polypeptides include polysaccharides such as sodium carboxymethylcellulose or methylcellulose, polyvinylpyrrolidone, polyacrylates, polyoxyethylene-polyoxypropylene-block polymers, PEG, and wood wax alcohols.

Examples of stabilizer include an amino acid, such as for instance, glycine; or an oligosaccharide, such as for example, sucrose, tetralose, lactose or a dextran. Alternatively, the stabilizer may be a sugar alcohol, such as for instance, mannitol; or a combination thereof.

The salt or buffering agent may be any salt or buffering agent, such as for example, sodium chloride, or sodium/potassium phosphate, respectively. Preferably, the buffering agent maintains the pH of the pharmaceutical composition in the range of about 5.5 to about 7.5. The salt and/or buffering agent is also useful to maintain the osmolality at a level suitable for administration to a human or an animal.

The peptides used for *in vivo* administration should be sterile. This may be accomplished by filtration through sterile filtration membranes, prior to or following lyophilization and reconstitution. The peptides ordinarily will be stored in lyophilized form or in solution. Therapeutic peptide compositions generally are placed into a container having a sterile access port, for example, an intravenous solution bag or vial having a stopper pierceable by a hypodermic injection needle.

The peptide for the use of the invention may be provided with an article of manufacture or a commercial package or kit, such as an FDA approved kit, which may comprise: a container, a label on the container and a composition comprising one or more unit dosage forms of the peptides of the present invention as active agent. The kit may be accompanied by instructions for dosage, administration and indications to be treated. The instructions may indicate that the composition is effective for, *inter alia,* protecting against protecting a subject against reperfusion injury.

An "effective amount" or a "therapeutically effective amount" refers to an amount effective, at dosages and for periods of time necessary, to achieve the desired therapeutic result. A therapeutically effective amount of the peptides noted herein may vary according to factors such as the disease state, age, sex, and weight of the individual, and the ability of the compound to elicit a desired response in the individual. Dosage regimens may be adjusted to provide the optimum therapeutic response. A therapeutically effective amount is also one in which any toxic or detrimental effects of the compound are outweighed by the therapeutically beneficial effects. A "prophylactically effective amount" refers to an amount effective, at dosages and for periods of time necessary, to achieve the desired prophylactic result,. A prophylactically effective amount can be determined as described above for the therapeutically effective amount. For any particular subject, specific dosage regimens may be adjusted over time according to the individual need and the professional judgment of the person administering or supervising the administration of the compositions.

For example, a therapeutically effective amount or effective dose of the peptides as defined herein (also referred to herein as "active compound") is an amount sufficient for in protecting a subject against reperfusion injury. The methods and/or assays for measuring such parameters are known to those of ordinary skill in the art.

The therapeutically effective amount of the invention will generally vary from 0.001 µg/kg to 100 mg/kg, more particularly from 0.01 µg/kg to 10 mg/kg, and even more particularly from 1 µg/kg to 1 mg/kg. Therapeutically effective amounts or effective doses that are outside this range but that have the desired therapeutic effect are also encompassed by the present invention.

In a further embodiment, the present UAG fragment for its use of the invention may be administered in combination with additional pharmacologically active substances or may be administered in combination with another therapeutic method. The combination may be in the form of a kit-in-part system, wherein the combined active substances may be used for simultaneous, sequential or separate administration.

### EXPERIMENTS AND DATA ANALYSIS

### UAG protects against ischemia-mediated functional impairment in skeletal muscle (Background Illustration)

Unilateral hind-limb ischemia was induced in C57BL/6J mice, and mice were treated daily with either saline, AG, or UAG, beginning at day 0. When a functional score was applied, it was observed that the damage was significantly higher in saline- and AG-treated groups than in the UAG-treated group as shown in Figure 1A. To assess whether these functional differences were due to differences in tissue reperfusion, the number of vessels was counted in ischemic muscles of treated animals. UAG-treated mice had a larger number of functional vessels compared to the other groups as shown in Figures 1B. Furthermore, ischemic muscles from AG- and saline-treated mice had a significantly lower capillary density than in contralateral control muscles from the same animals, whereas no such differences were observed in UAG-treated animals (Figure 1B). Analysis of tissue regeneration in gastrocnemius muscles revealed that muscles from UAG-treated mice contained an increased number of regenerating myofibers (Figures 1C and 1D). In addition, those mice had a reduced number of CD68-positive inflammatory cells (Figure 1E). No changes were observed in the numbers of regenerating fibers or CD68-positive inflammatory cells in normo-perfused muscles (Figures 1C, ID and 1E). Thus UAG but not AG appears to protect against ischemia-induced damage.

### UAG increases the number of Pax-7-positive cells (Background Illustration)

SMR *in vivo* depends on the expansion and differentiation of SCs³⁷ co-expressing Pax-7 and MyoD. Thus, the number of cells expressing both Pax-7 and MyoD was evaluated. At day 7, ischemic muscles from UAG-treated mice had an increased number of Pax-7/MyoD+ cells compared to ischemic muscles from AG-treated and control mice (Figure 2A). At day 21, scattered Pax-7/MyoD+ cells were still present in ischemic muscles from UAG- but not AG- or saline-treated mice (Figure 2A). SCs were also isolated and counted (Figure 2B). This *ex vivo* evaluation revealed a significantly increased number of Pax-7/MyoD/Myf5+ SCs in UAG-treated mice compared to AG- and saline-treated animals (Figures 2B, 2C and 2D). Consistent with previous reports indicating that SC proliferation depends on p38/MAPK activation^{36,37}, levels of phospho(p)-p38/MAPK protein were found to be higher in SCs from UAG-treated mice than in those from saline- or AG-treated groups (Figure 2C). Scattered Pax-7+/MyoD- cells were detected in normoperfused muscles in all groups. In addition, there were an increased number of myogenin-positive cells in ischemic muscles from UAG-treated mice (Figures 2E and 2F).

### UAG induces SOD-2 expression in SCs (Background Illustration)

Thiobarbituric acid reactive substances (TBARS) were first evaluated in ischemic muscles. Significant increases in the concentration of TBARS were observed in AG- and saline- but not UAG-treated muscles (Figure 3A). These data suggest that UAG may promote SC expansion by inducing an efficient antioxidant response. Since the mitochondria-specific antioxidant enzyme SOD-2 is known to be diminished in patients with PAD, the expression of SOD-2 expression and ROS content in SCs isolated from different groups were analyzed. It was found that ROS production was lower and SOD-2 protein expression higher in SCs from UAG-treated mice than from AG- or saline-treated animals (Figures 3B and 3C). Furthermore, double immunostaining (data not shown) of ischemic muscle from UAG-treated mice revealed an increased number of SOD-2/Pax-7+ cells compared to AG-treated mice and controls (Figure 3D). Finally, when UAG was administered to mice treated with BaCl₂, which is known to induce ROS-independent damage³⁸, regeneration of skeletal muscle was no longer observed (Figure 3E). This experiment and these data clearly demonstrate that UAG specifically acts on ROS-mediated damage, but not on toxic-mediated damage. UAG thus appears to influence SMR via an antioxidant effect.

Together, these data suggest that UAG may represent a defense mechanism against ROS and that diseases characterized by mitochondrial dysfunction and increased ROS generation, may likely also benefit from UAG treatment.

### UAG-induced SC cell-cycle entry is recapitulated in vitro even in mice lacking the entire ghrelin system (Background Illustration)

Primary SCs recovered from normo-perfused muscles were subjected to *in vitro* ischemia and evaluated for cell-cycle progression upon treatment. Again, only UAG challenge induced expression of Pax-7, MyoD (Figure 4A) and myogenin (Figure 4B), and increased levels of pp38/MAPK (Figure 4C). In addition, UAG challenge increased PCNA expression (Figure 4D) and the number of cells in S phase (Figure 4E). When examined under the same experimental conditions, ROS production was decreased (Figure 4F) and SOD-2 expression increased (Figure 4G) following UAG treatment. When SOD-2 was silenced in SCs using siRNA (Figure 5A) and subjected to *in vitro* ischemia, UAG did not protect SCs against ROS generation (Figure 5B), did not induce p38/MAPK phosphorylation (Figure 5D) and the cells did not undergo cell-cycle progression (Figure 5C). Moreover, addition of SB202190, an inhibitor of p38/MAPK phosphorylation, blocked cell-cycle entry and prevented MyoD and myogenin expression in SCs exposed to UAG (Figures 5E and 5F). These data indicate that, after ischemia, SOD-2 expression is important for UAG-induced p38/MAPK phosphorylation leading to cell-cycle entry in SCs.

To assess whether the effects of UAG on SMR and SC cell-cycle entry occurred through the classic ghrelin signaling pathway, mice lacking the GHSR1a and ghrelin genes³⁹ was analyzed. SCs from these double KO mice were subjected to *in vitro* ischemia in the presence of AG or UAG. Once again, only UAG promoted SC cell-cycle progression (Figure 5G) and induced p-p38/MAPK, Pax-7, MyoD, Myf5 and myogenin expression (Figures 5H and 5I). Moreover, unlike AG, UAG protected SCs from ROS generation and induced SOD-2 expression (Figures 5I and 5J). These findings, along with the failure to detect *in vivo* effects of AG, further support the possibility that UAG induces AG-independent activities via specific binding sites.

### miR-221 and miR-222 control UAG-induced SC cell-cycle entry by regulating the expression of p57^{Kip} (Background Illustration)

To address the mechanism through which UAG exerts its effects, the expression of miR-221 and miR-222, recently emerged as important regulators of myogenesis^{40,41}, was analyzed. Expression of miR-221 and miR-222 was significantly increased in SCs recovered from muscles of UAG-treated mice compared to controls (Figure 6A). The expression of p57^{Kip2}, a known target gene of miR-221/22241, was therefore analyzed and it was found that levels of p57^{Kip2} protein were reduced in SCs from UAG-treated mice (Figure 6B). Similar results were obtained when SCs recovered from normoperfused muscles were subjected to *in vitro* ischemia and UAG (Figures 6C and 6D). These data were confirmed by luciferase assay (Figure 6E). Furthermore, in loss-of-function experiments involving transfection of SCs with anti-miR-221/222 antago-miRs, UAG no longer had any effect on cell-cycle entry or expression of SOD-2, Pax-7, MyoD, or myogenin in SCs (data not shown). The observation that p-p38/MAPK levels were reduced under these experimental conditions (data not shown) provides further evidence that both miRs are important mediators of SC cell-cycle entry.

### miR-221/222 expression is modulated by oxidative stress and is important for SMR upon ischemia (Background Illustration)

Analysis of miR-221/222 expression in the *in vitro* model of ischemia following SOD-2 depletion (Figure 7A) revealed that SOD-2 knock-down prevents UAG-induced miR-221/222 expression (Figure 7B). Thus, miR-221/222 expression appears to be modulated by ROS generation. The *in vivo* role of miR-221/222 in SMR was analyzed by injection of pre-miR-221/222 in the herein discussed model. Under these conditions, pre-miR-221/222 injection led to lower damage scores and significant myofiber regeneration (Figures 7C, 7D and 7E) even in the absence of UAG. Furthermore, SCs recovered from those mice had high levels of miR-221/222 expression and increased levels of MyoD, myogenin and p-p38/MAPK protein (data not shown).

### UAG, fragments and analogs thereof protect C2C12 mouse muscular cell line from oxidative stress

The effect of UAG and UAG fragments on C2C12 mouse muscular cell line subjected to oxidative stress was assessed. In this analysis, oxidative stress was caused by hypoxia, AGEs, hyperglycemia or by H₂O₂. The results obtained demonstrate that UAG (comparative experiment), UAG (6-13) and UAG(6-13)cyclic have a protective effect on C2C12 cells against oxidative stress (Figure 8).

### VII) MATERIALS AND TECHNICAL PROTOCOLS

*Murine hind-limb ischemia model* - Male C57BL/6Jmice (Charles River Lab., Wilmington, MA, USA) were anesthetized and unilateral hind limb ischemia was induced as described⁴². The normo-perfused contralateral limb of each mouse was used as an internal control. After hind-limb ischemia, animals (18 mice per group) were treated by intra-peritoneal injection daily from 0 to day 21 with either saline, AG (100 µg/kg) or UAG (100 µg/kg). In selected experiments, mice received intramuscular injections of pre-miR oligonucleotides (5 mice/group). To induce toxic damage, 100 µl of 1% barium chloride (BaCl₂, Sigma Aldrich) was injected unilaterally into the hind limb (9 mice). Mice were treated according to European Guidelines and policies as approved by the University of Turin Ethical Committee. *In-vivo assessment of limb function* - Semiquantitative estimation (by repeated measures analyzed with ANOVA and Newman-Keuls Multiple Comparison test) of foot damage was performed serially using the following classification: 3 = dragging of foot (foot necrosis), 2 = no dragging but no plantar flexion (foot damage), 1 = plantar flexion but no toe flexion (toe damage), and 0=flexing the toes to resist gentle traction on the tail (no damage)⁴³.

*Histological and immunofluorescence (IF) analysis* - Gastrocnemius muscle sections from ischemic or normo-perfused limbs were stained with hematoxylin and eosin for histological analysis. The proportion of fibers with central nuclei (regenerating fibers) was measured by MetaMorph software (Life Sciences Research Imaging Systems) in the injured area and the cross-sectional areas of the fibers in the injured and non-injured areas. For IF analysis, muscle sections were processed as described previously⁴⁴. The number of cells expressing the indicated markers or CD31 positive vessels was evaluated as previously described³⁴.

*Cell cultures and in-vitro ischemia* - SCs were isolated from gastrocnemius muscles of C57BL/6J wild type mice subjected to ischemia or C57BL/6Jmice lacking the GHSR1a and ghrelin genes (10 mice, kind gift of Professor M. Tschöp)³⁹. To obtain SCs, muscle samples were subjected to enzymatic digestion as described⁴⁵. In selected experiments, SCs were recovered from normo-perfused muscles and subjected to in-vitro ischemia in presence of saline, AG (1µmol/L) or UAG (1µmol/L). In-vitro ischemia was induced by incubating cells in DMEM + 2% FCS at 5% CO₂/95% N₂ humidified atmosphere, yielding 1% O₂ concentrations for 24h¹⁸. The in-vitro ischemia was also performed in the presence of SB202190 (1µmol/L). *Cell-cycle progression and proliferation* - SC cell-cycle progression was evaluated by evaluating the percentage of PCNA-positive cells or by FACS analysis as previously described⁴⁶. The percentage of cells in each cell cycle phase was determined by ModFit LT software (Verity Software House. Inc, topsham, ME, USA). Celss proliferation was also assayed by evaluating the percentage of PCNA-positive cells by FACS analysis.

*Western Blot (WB) analysis* - Cells were lysed and protein detection was obtained as previously described⁴⁷. Cells were lysed (50 mmol/L Tris HCI [pH 8.3], 1% Triton X-100, 10 mmol/L PMSF, 100 U/ml aprotinin, 10 µmol/L leupeptin) and protein concentrations were obtained as previously described⁴⁷. Proteins (50 µg) were subjected to SDS-PAGE, transferred into nitrocellulose membrane, blotted with the indicated antibodies and revealed by chemiluminescence detection system (ECL). Densitometric analysis was used to calculate the differences in the fold induction of protein levels and normalized to tubulin, α actin or p38MAPK content. Values are reported as relative amount

*Oxidative stress measurement* - Intracellular ROS production was evaluated using DCF-DA (5-(and-6)-carboxy-2',7'-dichlorofluorescein diacetate, 0.5 Lmol/L final concentration) (Molecular Probe, Invitrogen) assay as previously described²³. The formation of TBARS was determined in muscles using the OXI-TEK kit (ZeptoMetrix Corp.) and a luminescence spectrometer (Bio-Rad Laboratories, Hercules, CA) with excitation set at 530 nm, emission at 550 nm to measure in-vivo oxidative stress levels³³.

*RNA isolation and quantitative real-time PCR (qRT-PCR) for miRNAs* - Total RNA was isolated using TRIzol reagent (Invitrogen) from SCs recovered from muscles of treated animals or from SCs subjected to in-vitro ischemia. miR-221/222 expression was evaluated by qRT-PCR as previously described⁴⁸. Loss-of-function experiments were performed in SCs transfected for 48h with anti-miRNA negative control, anti-miR-221/222 antagonists (Applied Biosystem, Foxter Cyto CA, USA), according to the manufacturer's instructions⁴⁸.

*SOD-2 silencing by small interfering RNAs (siRNA)* - To obtain SOD-2 inactivation, SCs were transiently transfected with siRNA for SOD-2 or with duplex siRNAs (Qiagen, Valencia, CA, USA) as previously described⁴⁴. Transfection was performed according to the manufacturer's instructions. Whole cell extracts were processed 48h after transfection. Cell viability was evaluated at the end of each experiment.

*Luciferase miRNA target reporter assay* - The luciferase reporter assay was performed using a construct generated by subcloning the PCR products amplified from the full-length 3'UTR of p57Kip2 as previously described⁴⁷.

*In-vivo gain of function analysis* - To evaluate the effects of miR-221/222 expression in-vivo, a combination of pre-miR-221/222 or pre-miR negative control (50 µl of 50 nM stock solution of pre-miR oligonucleotides into 12 µl of Optifect, Invitrogen) was injected directly into the ischemic gastrocnemius muscle of C57BL/6J mice. Pre-miRs or controls were administrated 3 times a week. At day 7, animals were sacrificed and tissues were recovered and processed as described above for histological analysis. SCs were also isolated and evaluated by WB for the indicated markers and by qRT-PCR for miRNA expression.

*Statistical analysis* - Between-group comparisons were carried out by *t* test. Comparisons between 3 or more groups were performed by one-way ANOVA and significance was evaluated using the Newman-Keuls multi-comparison post hoc test. The cutoff for statistical significance was set at P < 0.05. All statistical analyses were carried out with Graph Pad Prism version 5.04 (Graph Pad Software, Inc, USA).

*Oxidative stress measurement* - Kinetic analysis of ROS production was evaluated by using DCF-DA (5-(and-6)-carboxy-2',7'-dichlorofluorescein diacetate, 0,5 µmol/L final concentration) (Molecular Probe, Invitrogen) assay. C2C12 cells were cultured with 400 µg/ml Advanced Glycated End-product (AGE) or 25 mM glucose (high glucose HG) for 48h, H₂O₂ (100 µM) for 2h. In parallel experiments C2C12 cells were subjected to hypoxia (DMEM + 2% FCS in a 5% CO₂-95% N₂ humidified atmosphere, yielding to 1% O₂ concentrations for 24h). UAG (1µmol/L) AZP 531 (1µmol/L) or AZP-502 (1 µmol/L) was added where indicated.

### REFERENCE LIST:

1. Kojima M, Hosoda H, Date Y, Nakazato M, Matsuo H and. Kangawa K; Ghrelin is a growth-hormone-releasing acylated peptide from stomach, Nature 402:656-660 (1999).
2. Gnanapavan S, Kola B, Bustin SA, Morris DG, McGee P, Fairclough P, Bhattacharya S, Carpenter R, Grossman AB and Korbonits M; The tissue distribution of the mRNA of ghrelin and subtypes of its receptor, GHS-R, in humans, J. Clin. Endocrinol. Metab. 87:2988-2991 (2002).
3. Howard AD, Feighner SD, Cully DF, Arena JP, Liberator PL, Rosenblum Cl, Hamelin M, Hreniuk DL, Palyha OC, Anderson J, Paress PS, Diaz C, Chou M, Liu KK, McKee K-K, Pong S-S, Chaung L-Y, Elbrecht A, Dashkevicz M, Heavens R, Rigby M, Sirinathsinghji D, Dean DC, Melillo DG, Patchett AA, Nargund R, Griffin PR, DeMartino JA, Gupta SK, Schaeffer JA, Smith RG, Van der Ploeg LHT; A receptor in pituitary and hypothalamus that functions in growth hormone release. Science 273:974-977 (1996).
4. Ariyasu H, Takaya K, Tagami T, Ogawa Y, Hosoda K, Akamizu T, Suda M, Koh T, Natsui K, Toyooka S, Shirakami G, Usui T, Shimatsu A, Doi K, Hosoda H, Kojima M, Kangawa K, Nakao K.; Stomach is a major source of circulating ghrelin, and feeding state determines plasma ghrelin-like immunoreactivity levels in humans. J Clin Endocrinol Metab. Oct;86(10):4753-8 (2001)
5. Date Y, Murakami N, Toshinai K, Matsukura S, Niijima A, Matsuo H, Kangawa K, Nakazato M. The role of the gastric afferent vagal nerve in ghrelin-induced feeding and growth hormone secretion in rats. Gastroenterology 123(4):1120-1128, 2002.
6. Kojima M, Hosoda H, Date Y, Nakazato M, Matsuo H, Kangawa K. Ghrelin is a growth-hormone-releasing acylated peptide from stomach. Nature 402(6762):656-660, 1999.
7. Tschop M, Smiley DL, Heiman ML. Ghrelin induces adiposity in rodents. Nature 407(6806):908-913, 2000.
8. Broglio F, Gianotti L, Destefanis S, Fassino S, Abbate Daga G, Mondelli V, Lanfranco F, Gottero C, Gauna C, Hofland L, Van Der Lely AJ, Ghigo E. The endocrine response to acute ghrelin administration is blunted in patients with anorexia nervosa, a ghrelin hypersecretory state. Clin Endocrinol (Oxf) 60(5):592-599, 2004.
9. Tong J, Prigeon RL, Davis HW, Bidlingmaier M, Kahn SE, Cummings DE, Tschop MH, D'Alessio D. Ghrelin suppresses glucose-stimulated insulin secretion and deteriorates glucose tolerance in healthy humans. Diabetes Sep;59(9):2145-51 (2010).
10. van der Lely AJ, Tschop M, Heiman ML, Ghigo E; Biological, physiological, pathophysiological, and pharmacological aspects of ghrelin. Endocr Rev 25:426-457 (2004).
11. Asakawa A, Inui A, Fujimiya M, Sakamaki R, Shinfuku N, Ueta Y, Meguid MM, Kasuga M. Stomach regulates energy balance via acylated ghrelin and desacyl ghrelin. Gut. Jan;54(1):18-24 (2005).
12. Broglio F, Gottero C, Prodam F, Gauna C, Muccioli G, Papotti M, Abribat T, Van Der Lely AJ, Ghigo E. Non-acylated ghrelin counteracts the metabolic but not the neuroendocrine response to acylated ghrelin in humans. J Clin Endocrinol Metab. Jun;89(6):3062-5 (2004).
13. Gauna C, Meyler FM, Janssen JA, Delhanty PJ, Abribat T, van Koetsveld P, Hofland LJ, Broglio F, Ghigo E, van der Lely AJ. Administration of acylated ghrelin reduces insulin sensitivity, whereas the combination of acylated plus unacylated ghrelin strongly improves insulin sensitivity. J Clin Endocrinol Metab. Oct;89(10):5035-42 (2004).
14. Kiewiet RM, van Aken MO, van der Weerd K, Uitterlinden P, Themmen AP, Hofland LJ, de Rijke YB, Delhanty PJ, Ghigo E, Abribat T, van der Lely AJ. Effects of acute administratio of acylated and unacylated ghrelin on glucose and insulin concentratios in morbidly obese subjects without overt diabetes. Eur J Endocrinol 161:567-573 (2009).
15. Gauna C, Delhanty PJD, Hofland LJ, Janssen J, Broglio F, Ross RJM, Ghigo E, van der Lely AJ. Ghrelin stimulates, whereas des-octanoyl ghrelin inhibits, glucose output by primary hepatocytes. Journal of Clinical Endocrinology and Metabolism 90:1055-1060 (2005).
16. Gauna C, Kiewiet RM, Janssen JA, van de Zande B, Delhanty PJ, Ghigo E, Hofland LJ, Themmen AP, van der Lely AJ. Unacylated ghrelin acts as a potent insulin secretagogue in glucose-stimulated conditions. Am J Physiol Endocrinol Metab 293:E697-704 (2007).
17. Zhao W, Diz DI, Robbins ME; Oxidative damage pathways in relation to normal tissue injury. Br J Radiol. 80:S23-31 (2007).
18. Filigheddu N, Gnocchi VF, Coscia M, et al. Ghrelin and des-acyl ghrelin promote differentiation and fusion of C2C12 skeletal muscle cells. Mol Biol Cell. 2007;18:986-994.
19. Baldanzi G, Filigheddu N, Cutrupi S, et al. Ghrelin and des-acyl ghrelin inhibit cell death in cardiomyocytes and endothelial cells through ERK1/2 and PI 3-kinase/AKT. J Cell Biol .2002;159:1029-1037.
20. Gauna C, Delhanty PJ, Hofland LJ, Janssen JA, Broglio F, Ross RJ, Ghigo E, van der Lely AJ. Ghrelin stimulates, whereas des-octanoyl ghrelin inhibits, glucose output by primary hepatocytes. J Clin Endocrinol Metab. 2005;90:1055-1060.
21. Soares JB, Leite-Moreira AF. Ghrelin, des-acyl ghrelin and obestatin: three pieces of the same puzzle. Peptides. 2008;29:255-1270.
22. Lear PV, Iglesias MJ, Feijoo-Bandin S, Rodriguez-Penas D, Mosquera-Leal A, Garcia-Rua V, Gualillo O, Ghe C, Arnoletti E, Muccioli G., Dieguez C, Gonzalez-Juanatey JR, Lago F. Des-acyl ghrelin has specific binding sites and different metabolic effects from ghrelin in cardiomyocytes. Endocrinology. 2010;151:3286-3298.
23. Togliatto G, Trombetta A, Dentelli P, Baragli A, Rosso A, Granata R, Ghigo D, Pegoraro L, Ghigo E, Brizzi MF. Unacylated ghrelin rescues endothelial progenitor cell function in individuals with type 2 diabetes. Diabetes. 2010;59:1016-1025.
24. Granata R, Settanni F, Julien M, Nano R, Togliatto G, Trombetta A, Gallo D, Piemonti L, Brizzi MF, Abribat T, van Der Lely AJ, Ghigo E. Des-acyl ghrelin fragments and analogues promote survival of pancreatic β-cells and human pancreatic islets and prevent diabetes in streptozotocin-treated rats. J Med Chem. 2012;55:2585-2596.
25. Weitz JI, Byrne J, Clagett GP, Farkouh ME, Porter JM, Sackett DL, Strandness DE Jr, Taylor LM. Diagnosis and treatment of chronic arterial insufficiency of the lower extremities: a critical review. Circulation. 1996;94:3026-3049.
26. Gray BH, Conte MS, Dake MD, Jaff MR, Kandarpa K, Ramee SR, Rundback J, Waksman R, American Heart Association Writing Group 7. Atherosclerotic Peripheral Vascular Disease Symposium II: lower-extremity revascularization: state of the art. Circulation. 2008;118:2864-2872.
27. Cieri E, Lenti M, De Rango P, Isernia G, Marucchini A, Cao P. Functional ability in patients with critical limb ischaemia is unaffected by successful revascularisation. Eur J Vasc Endovasc Surg. 2011;41:256-263.
28. Pipinos II, Judge AR, Selsby JT, Zhu Z, Swanson SA, Nella AA, Dodd SL. The myopathy of peripheral arterial occlusive disease: Part 2. Oxidative stress, neuropathy, and shift in muscle fiber type. Vasc Endovascular Surg. 2008;42:101-112.
29. Bhat HK, Hiatt WR, Hoppel CL, Brass EP. Skeletal muscle mitochondrial DNA injury in patients with unilateral peripheral arterial disease. Circulation. 1999;99:807-812.
30. Finkel T. Radical medicine: treating ageing to cure disease. Nat Rev Mol Cell Biol. 2005;6:971-976.
31. Madamanchi NR, Runge MS. Mitochondrial Dysfunction in Atherosclerosis. Circ. Res. 2007;100:460-473.
32. Zaccagnini G, Martelli F, Fasanaro P, Magenta A, Gaetano C, Di Carlo A, Biglioli P, Giorgio M, Martin-Padura I, Pelicci PG, Capogrossi MC. p66ShcA modulates tissue response to hindlimb ischemia. Circulation. 2004;109:2917-2923.
33. Zaccagnini G, Martelli F, Magenta A, Cencioni C, Fasanaro P, Nicoletti C, Biglioli P, Pelicci PG, Capogrossi MC. p66(ShcA) and oxidative stress modulate myogenic differentiation and SMR after hind limb ischemia. J Biol Chem. 2007;282:31453-31459.
34. Pedersen BL, Baekgaard N, Quistorff B. Muscle mitochondrial function in patients with type 2 diabetes mellitus and peripheral arterial disease: implications in vascular surgery. Eur J Vasc Endovasc Surg. 2009;38:356-364.
35. Loffredo L, Marcoccia A, Pignatelli P, Andreozzi P, Borgia MC, Cangemi R, Chiarotti F, Violi F. Oxidative-stress-mediated arterial dysfunction in patients with peripheral arterial disease Eur Heart J. 2007;28:608-612.
36. Jones NC, Tyner KJ, Nibarger L, Stanley HM, Cornelison DD, Fedorov YV, Olwin BB. The p38alpha/beta MAPK functions as a molecular switch to activate the quiescent satellite cell. J Cell Biol.2005;169:105-116.
37. Troy A, Cadwallader AB, Fedorov Y, Tyner K, Tanaka KK, Olwin BB. Coordination of satellite cell activation and self-renewal by Par-complex-dependent asymmetric activation of p38α/β MAPK. Cell Stem Cell. 2012;11: 541-553.
38. Lu H, Huang D, Ransohoff RM, Zhou L. Acute skeletal muscle injury: CCL2 expression by both monocytes and injured muscle is required for repair. FASEB J. 2011;25:3344-3355.
39. Kirchner H, Tong J, Tschop MH, Pfluger PT. Ghrelin and PYY in the regulation of energy balance and metabolism: lessons from mouse mutants. Am J Physiol Endocrinol Metab. 2010;298:E909-919.
40. Cardinali B, Castellani L, Fasanaro P, Basso A, Alema S, Martelli F, Falcone G. Microrna-221 and microrna-222 modulate differentiation and maturation of skeletal muscle cells. PLoS One. 2009;4:e7607.
41. Greco S, Perfetti A, Fasanaro P, Cardani R, Capogrossi MC, Meola G, Martelli F. Deregulated microRNAs in myotonic dystrophy type 2. PLoS One. 2012;7:e39732.
42. Hellingman AA, Bastiaansen AJ, de Vries MR, Seghers L, Lijkwan MA, Lowik CW, Hamming JF, Quax PH. Variations in surgical procedures for hind limb ischaemia mouse models result in differences in collateral formation. Eur J Vasc Endovasc Surg. 2010;40:796-803.
43. Bosch-Marce M, Okuyama H, Wesley JB, et al. Effects of aging and hypoxia-inducible factor-1 activity on angiogenic cell mobilization and recovery of perfusion after limb ischemia. Circ Res. 2007;101:1310-1318.
44. Zeoli A, Dentelli P, Rosso A, Togliatto G, Trombetta A, Damiano L, di Celle PF, Pegoraro L, Altruda F, Brizzi MF. Interleukin-3 promotes expansion of hemopoietic-derived CD45+ angiogenic cells and their arterial commitment via STAT5 activation. Blood. 2008;112:350-361.
45. Musaro A, Barberi L. Isolation and culture of mouse satellite cells. Methods Mol Biol. 2010;633:101-111.
46. Dentelli P, Rosso A, Olgasi C, Camussi G, Brizzi MF. IL-3 is a novel target to interfere with tumor vasculature. Oncogene. 2011;30:4930-4940.
47. Togliatto G, Trombetta A, Dentelli P, Rosso A, Brizzi MF. MIR221/MIR222-driven posttranscriptional regulation of P27KIP1 and P57KIP2 is crucial for high-glucose- and AGEmediated vascular cell damage. Diabetologia. 2011;54:1930-1940.
48. Dentelli P, Rosso A, Orso F, Olgasi C, Taverna D, Brizzi MF. microRNA-222 controls neovascularization by regulating signal transducer and activator of transcription 5A expression. Arterioscler Thromb Vasc Biol. 2010;30:1562-1568.
49. Delhanty PJ, Neggers SJ, van der Lely AJ. Mechanisms in endocrinology: Ghrelin: the differences between acyl- and des-acyl ghrelin. Eur J Endocrinol 2012 Nov;167(5):601-8.
50. Granata R, Settanni F, Julien M, Nano R, Togliatto G, Trombetta A, Gallo D, Piemonti L, Brizzi MF, Abribat T, van Der Lely AJ, Ghigo E. Des-Acyl ghrelin fragments and analogues promote survival of pancreatic b-cells and human pancreatic islets and prevent diabetes in streptozotocin-treated rats. J. Med. Chem. 2012, 55, 2585-2596.

## Claims

1. A fragment of unacylated ghrelin and/or pharmaceutically acceptable salts thereof wherein the fragment consists of amino acid sequence SEQ ID NO: 6 and/or pharmaceutically acceptable salts thereof, for its use in protecting a subject against reperfusion injury.

2. The fragment for its use as defined in claim 1, wherein it is in a cyclized form.

3. The fragment for its use as defined in claim 2, wherein it consists of amino acid sequence SEQ ID NO: 25.

4. The fragment for its use as defined in any one of claims 1 to 3, wherein the fragment comprises one or more of a linker moiety and a leader sequence.

5. The fragment for its use as defined in claim 4, wherein the leader sequence is a mitochondrial leader sequence.

6. The fragment for its use as defined in any one of claims 1 to 3, wherein it is in a pro-protein format.

7. The fragment for its use as defined in any one of claims 1 to 6, wherein it modulates cellular levels of superoxide-dismutase-2 (SOD-2).

8. The fragment for its use as defined in claim 7, wherein the modulation of cellular levels of SOD-2 includes increasing cellular levels or expression of SOD-2.

9. The fragment for its use as defined in any one of claims 1 to 8, wherein it is administered in an effective amount between 0.001 µg/kg and 100 mg/kg.

10. The fragment for its use as defined in any one of claims 1 to 9, wherein the subject suffers from ischemia.

11. The fragment for its use as defined in any one of claims 1 to 9, wherein the subject suffers from a stroke.

12. The fragment for its use as defined in any one of claims 1 to 9, for protecting a subject from reperfusion injury following organ transplantation.

13. The fragment for its use as defined in any one of claims 1 to 9, for preventing ischemia-reperfusion injury in the subject.

## Patentansprüche

1. Fragment von nicht-acyliertem Ghrelin und/oder pharmazeutisch annehmbare Salze davon, wobei das Fragment aus der Aminosäuresequenz SEQ ID NO: 6 und/oder pharmazeutisch annehmbaren Salzen davon besteht, zur Verwendung zum Schutz eines Individuums gegen Reperfusionsschaden.

2. Fragment zur Verwendung nach Anspruch 1, wobei es in einer zyklisierten Form vorliegt.

3. Fragment zur Verwendung nach Anspruch 2, wobei es aus der Aminosäuresequenz SEQ ID NO: 25 besteht.

4. Fragment zur Verwendung nach einem der Ansprüche 1 bis 3, wobei das Fragment eines oder mehrere von einer Linkereinheit und einer Leadersequenz umfasst.

5. Fragment zur Verwendung nach Anspruch 4, wobei die Leadersequenz eine mitochondriale Leadersequenz ist.

6. Fragment zur Verwendung nach einem der Ansprüche 1 bis 3, wobei es in einem Pro-Protein-Format vorliegt.

7. Fragment zur Verwendung nach einem der Ansprüche 1 bis 6, wobei es zelluläre Superoxid-Dismutase-2(SOD2)-Spiegel moduliert.

8. Fragment zur Verwendung nach Anspruch 7, wobei die Modulation der zellulären SOD2-Spiegeln das Erhöhen der zellulären SOD2-Spiegeln oder SOD2-Expression beinhaltet.

9. Fragment zur Verwendung nach einem der Ansprüche 1 bis 8, wobei es in einer wirksamen Menge zwischen 0,001 µg/kg und 100 mg/kg verabreicht wird.

10. Fragment zur Verwendung nach einem der Ansprüche 1 bis 9, wobei das Individuum unter Ischämie leidet.

11. Fragment zur Verwendung nach einem der Ansprüche 1 bis 9, wobei das Individuum unter einem Schlaganfall leidet.

12. Fragment zur Verwendung nach einem der Ansprüche 1 bis 9, zum Schutz eines Individuums vor Reperfusionsschaden nach Organtransplantation.

13. Fragment zur Verwendung nach einem der Ansprüche 1 bis 9, zum Verhinderung von Ischämie-Reperfusionsschaden beim Individuum.

## Revendications

1. Fragment de ghréline non acylée et/ou de sels pharmaceutiquement acceptables de celle-ci, dans lequel le fragment est constitué de la séquence d'acides aminés SEQ ID NO : 6 et/ou de sels pharmaceutiquement acceptables de celle-ci, pour leur utilisation dans la protection d'un sujet contre une lésion de reperfusion.

2. Fragment pour son utilisation selon la revendication 1, dans lequel il est sous une forme cyclisée.

3. Fragment pour son utilisation selon la revendication 2, dans lequel il est constitué de la séquence d'acides aminés SEQ ID NO: 25.

4. Fragment pour son utilisation selon l'une quelconque des revendications 1 à 3, dans lequel le fragment comprend un ou plusieurs fragment(s) de liaison et une séquence de tête.

5. Fragment pour son utilisation selon la revendication 4, dans lequel la séquence de tête est une séquence de tête mitochondriale.

6. Fragment pour son utilisation selon l'une quelconque des revendications 1 à 3, dans lequel il est sous un format de pro-protéine.

7. Fragment pour son utilisation selon l'une quelconque des revendications 1 à 6, dans lequel il module les niveaux cellulaires de superoxyde dismutase 2 (SOD-2).

8. Fragment pour son utilisation selon la revendication 7, dans lequel la modulation des niveaux cellulaires de SOD-2 comprend une augmentation des niveaux cellulaires ou de l'expression de SOD-2.

9. Fragment pour son utilisation selon l'une quelconque des revendications 1 à 8, dans lequel il est administré en une quantité efficace comprise entre 0,001 µg/kg et 100 mg/kg.

10. Fragment pour son utilisation selon l'une quelconque des revendications 1 à 9, dans lequel le sujet souffre d' ischémie.

11. Fragment pour son utilisation selon l'une quelconque des revendications 1 à 9, dans lequel le sujet souffre d'un accident vasculaire.

12. Fragment pour son utilisation selon l'une quelconque des revendications 1 à 9, pour protéger un sujet d'une lésion de reperfusion suite à une greffe d'organe.

13. Fragment pour son utilisation selon l'une quelconque des revendications 1 à 9, pour prévenir une lésion de reperfusion-ischémie chez le sujet.


### SEQUENCE LISTING

<110> ALIZE PHARMA SAS
   Maria Felice Brizzi
   Ezio Ghigo
<120> USE OF UNACYLATED GHRELIN, FRAGMENTS AND ANALOGS THEREOF AS
   ANTIOXIDANT
<130> B2013-011-WO
<150> 61/837,723
   <151> 2013-06-21
<160> 30
<170> PatentIn version 3.5
<210> 1
   <211> 28
   <212> PRT
   <213> Homo sapiens
<400> 1
   Gly Ser Ser Phe Leu Ser Pro Glu His Gln Arg Val Gln Gln Arg Lys
   1               5                   10                  15
   Glu Ser Lys Lys Pro Pro Ala Lys Leu Gln Pro Arg
               20                  25
<210> 2
   <211> 14
   <212> PRT
   <213> Homo sapiens
<400> 2
   Gly Ser Ser Phe Leu Ser Pro Glu His Gln Arg Val Gln Gln
   1               5                   10
<210> 3
   <211> 18
   <212> PRT
   <213> Homo sapiens
<400> 3
   Gly Ser Ser Phe Leu Ser Pro Glu His Gln Arg Val Gln Gln Arg Lys
   1               5                   10                  15
   Glu Ser
<210> 4
   <211> 5
   <212> PRT
   <213> Homo sapiens
<400> 4
   Gly Ser Ser Phe Leu
   1               5
<210> 5
   <211> 12
   <212> PRT
   <213> Homo sapiens
<400> 5
   Glu Ser Lys Lys Pro Pro Ala Lys Leu Gln Pro Arg
   1               5                   10
<210> 6
   <211> 8
   <212> PRT
   <213> Homo sapiens
<400> 6
   Ser Pro Glu His Gln Arg Val Gln
   1 5
<210> 7
   <211> 6
   <212> PRT
   <213> Homo sapiens
<400> 7
   Glu His Gln Arg Val Gln
   1 5
<210> 8
   <211> 5
   <212> PRT
   <213> Homo sapiens
<400> 8
   Glu His Gln Arg Val
   1 5
<210> 9
   <211> 13
   <212> PRT
   <213> Homo sapiens
<400> 9
   Ser Pro Glu His Gln Arg Val Gln Gln Arg Lys Glu Ser
   1 5 10
<210> 10
   <211> 4
   <212> PRT
   <213> Homo sapiens
<400> 10
   Glu His Gln Arg
   1
<210> 11
   <211> 4
   <212> PRT
   <213> Homo sapiens
<400> 11
   His Gln Arg Val
   1
<210> 12
   <211> 8
   <212> PRT
   <213> Homo sapiens
<400> 12
   Ser Pro Asp His Gln Arg Val Gln
   1 5
<210> 13
   <211> 8
   <212> PRT
   <213> Homo sapiens
<400> 13
   Ser Pro Glu His Gln Lys Val Gln
   1 5
<210> 14
   <211> 8
   <212> PRT
   <213> Homo sapiens
<400> 14
   Gly Pro Glu His Gln Arg Val Gln
   1 5
<210> 15
   <211> 8
   <212> PRT
   <213> Homo sapiens
<400> 15
   Ala Pro Glu His Gln Arg Val Gln
   1 5
<210> 16
   <211> 8
   <212> PRT
   <213> Homo sapiens
<400> 16
   Ser Ala Glu His Gln Arg Val Gln
   1 5
<210> 17
   <211> 8
   <212> PRT
   <213> Homo sapiens
<400> 17
   Ser Pro Ala His Gln Arg Val Gln
   1 5
<210> 18
   <211> 8
   <212> PRT
   <213> Homo sapiens
<400> 18
   Ser Pro Glu Ala Gln Arg Val Gln
   1 5
<210> 19
   <211> 8
   <212> PRT
   <213> Homo sapiens
<400> 19
   Ser Pro Glu His Ala Arg Val Gln
   1 5
<210> 20
   <211> 8
   <212> PRT
   <213> Homo sapiens
<400> 20
   Ser Pro Glu His Gln Ala Val Gln
   1 5
<210> 21
   <211> 8
   <212> PRT
   <213> Homo sapiens
<400> 21
   Ser Pro Glu His Gln Arg Ala Gln
   1 5
<210> 22
   <211> 8
   <212> PRT
   <213> Homo sapiens
<400> 22
   Ser Pro Glu His Gln Arg Val Ala
   1 5
<210> 23
   <211> 8
   <212> PRT
   <213> Homo sapiens
<220>
   <221> MOD_RES
   <222> (1)..(1)
   <223> ACETYLATION
<400> 23
   Ser Pro Glu His Gln Arg Val Gln
   1               5
<210> 24
   <211> 8
   <212> PRT
   <213> Homo sapiens
<220>
   <221> MOD_RES
   <222> (1)..(1)
   <223> ACETYLATION
<220>
   <221> MISC_FEATURE
   <222> (2)..(2)
   <223> D-amino acid
<400> 24
   Ser Pro Glu His Gln Arg Val Gln
   1               5
<210> 25
   <211> 8
   <212> PRT
   <213> Homo sapiens
<220>
   <221> MISC_FEATURE
   <222> (1)..(8)
   <223> Cyclic
<400> 25
   Ser Pro Glu His Gln Arg Val Gln
   1               5
<210> 26
   <211> 8
   <212> PRT
   <213> Homo sapiens
<220>
   <221> MISC_FEATURE
   <222> (3)..(6)
   <223> Cyclic
<400> 26
   Ser Pro Glu His Gln Lys Val Gln
   1               5
<210> 27
   <211> 8
   <212> PRT
   <213> Homo sapiens
<220>
   <221> MOD_RES
   <222> (1)..(1)
   <223> ACETYLATION
<220>
   <221> MISC_FEATURE
   <222> (3)..(6)
   <223> Cyclic
<400> 27
   Ser Pro Glu His Gln Lys Val Gln
   1               5
<210> 28
   <211> 8
   <212> PRT
   <213> Homo sapiens
<220>
   <221> MOD_RES
   <222> (1)..(1)
   <223> ACETYLATION
<400> 28
   Ser Pro Glu His Gln Lys Val Gln
   1               5
<210> 29
   <211> 3
   <212> PRT
   <213> Homo sapiens
<400> 29
   His Gln Arg
   1
<210> 30
   <211> 14
   <212> PRT
   <213> Homo sapiens
<400> 30
   Gln Gln Val Arg Gln His Glu Pro Ser Leu Phe Ser Ser Gly
   1               5                   10